# EUROPEAN PATENT APPLICATION

(11) **EP 4 375 272 A1**
(43) Date of publication of application: **29.05.2024**
(21) Application number: 23212307.5
(22) Date of filing: 27.11.2023
(51) Int. Cl.: C07D 209/82, C07D 251/24, C07D 307/91, C07D 311/86, C07D 333/76, C07D 335/16, C07D 403/04, C07D 403/10, C07D 405/14, C07D 409/04, C07D 409/10, C07D 409/14, C07D 487/04, C09K 11/06, H10K 50/11, H10K 85/60

(54) **OLED COMPRISING A COMBINATION OF TWO HOST COMPOUNDS IN THE LIGHT EMITTING LAYER**

(30) Priority: 28.11.2022 JP 2022189343
(71) Applicant: CANON KABUSHIKI KAISHA, Tokyo 146-8501 (JP)
(72) Inventor: IWAWAKI, Hironobu, Tokyo,, 146-8501 (JP); MIYASHITA, Hirokazu, Tokyo,, 146-8501 (JP); YAMADA, Naoki, Tokyo,, 146-8501 (JP)
(74) Representative: TBK

(57) **Abstract**

The present invention provides a composition containing an organic compound AB and an organic compound AA different from the organic compound AB. The organic compound AB is represented by the following general formula [1] and has a fused polycyclic structure A with two or more rings and a ring structure B different from the fused polycyclic structure A. The organic compound AA has the fused polycyclic structure A and is represented by the following general formula [2].

A-B _{[1]}

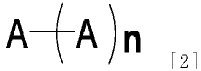

In general formulae [1] and [2], each of the fused polycyclic structures A may have, as a substituent, a deuterium atom, a substituted or unsubstituted alkyl group, a substituted or unsubstituted aryl group, or a substituted or unsubstituted heteroaryl group.

## Description

### BACKGROUND OF THE INVENTION

### Field of the Invention

The present invention relates to an organic compound and an organic light-emitting device including the organic compound.

### Description of the Related Art

Organic light-emitting devices (hereinafter, also referred to as "organic electroluminescent devices" or "organic EL devices") are electronic devices each including a pair of electrodes and an organic compound layer disposed between these electrodes. The injection of electrons and holes from these pairs of electrodes generates excitons in the light-emitting organic compound in the organic compound layer, and when the excitons return to the ground state, the organic light-emitting device emits light.

Recently, organic light-emitting devices have made remarkable progress and have achieved low-driving voltage, various emission wavelengths, and fast response time. The use of this has resulted in thinner and lighter light-emitting devices.

To further expand the applications of organic light-emitting devices, a further improvement in operating lifetimes of organic light-emitting devices is required. It is known that one way to improve the operating lifetime of organic light-emitting devices is to reduce the driving voltage.

Japanese Patent Laid-Open No. 2021-027169 describes an organic light-emitting device having excellent quantum efficiency and a low driving voltage by using a polycyclic aromatic compound in which a plurality of aromatic rings are fused and using a multimer thereof.

Japanese Patent Laid-Open No. 2021-027169 discloses that the use of a multimer improves the performance of an organic light-emitting device. The relationship between a monomer and the multimer is not described. There is room for a further improvement in the operating lifetime of an organic light-emitting device using a multimer.

### SUMMARY OF THE INVENTION

The present invention provides a light-emitting composition that improves the characteristics of an organic light-emitting device by using the relationship between a monomer and a multimer.

The present invention in its first aspect provides a composition as specified in claims 1 to 10.

Further features of the present invention will become apparent from the following description of exemplary embodiments with reference to the attached drawings.

### BRIEF DESCRIPTION OF THE DRAWINGS

Fig. 1A is a schematic cross-sectional view of an example of a pixel of a display apparatus according to an embodiment of the present invention. Fig. 1B is a schematic cross-sectional view of an example of a display apparatus including organic light-emitting devices according to an embodiment of the present invention.
Fig. 2 is a schematic view of an example of a display apparatus according to an embodiment of the present invention.
Fig. 3A is a schematic view of an example of an image pickup apparatus according to an embodiment of the present invention. Fig. 3B is a schematic view of an example of an electronic apparatus according to an embodiment of the present invention.
Fig. 4A is a schematic view of an example of a display apparatus according to an embodiment of the present invention. Fig. 4B is a schematic view of an example of a foldable display apparatus.
Fig. 5A is a schematic view of an example of a lighting apparatus according to an embodiment of the present invention. Fig. 5B is a schematic view of an example of an automobile including an automotive lighting unit according to an embodiment of the present invention.
Fig. 6A is a schematic view of an example of a wearable device according to an embodiment of the present invention. Fig. 6B is a schematic view of an example of a wearable device according to an embodiment of the present invention, the wearable device including an image pickup apparatus.
Fig. 7A is a schematic view of an image-forming apparatus according to an embodiment of the present invention. Figs. 7B and 7C are schematic views each illustrating a configuration of an exposure light source including multiple light-emitting portions arranged on a long substrate.

### DESCRIPTION OF THE EMBODIMENTS

A composition according to an embodiment of the present invention uses the relationship between a monomer and a multimer to expand a recombination zone. Thus, the use of the composition in an organic light-emitting device results in a prolonged operating lifetime of the organic light-emitting device.

A composition according to an embodiment of the present invention contains an organic compound AB and an organic compound AA different from the organic compound AB. The organic compound AB is represented by the following general formula [1] and has a fused polycyclic structure A with two or more rings and a ring structure B different from the fused polycyclic structure A. The organic compound AA has the fused polycyclic structure A and is represented by the following general formula [2].

A-B [1]

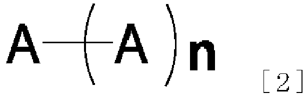

In general formulae [1] and [2], each of the fused polycyclic structures A may have, as a substituent, a deuterium atom, a substituted or unsubstituted alkyl group, a substituted or unsubstituted aryl group, or a substituted or unsubstituted heteroaryl group. However, these aryl and heteroaryl groups each have a structure containing two or more rings. Thus, for example, a phenyl group and a pyridyl group are excluded.

In this specification, the alkyl group may be an alkyl group, for example, having 1 to 20 carbon atoms, and may be linear, branched, or cyclic. The number of carbon atoms may be 1 or more and 8 or less, or may be 1 or more and 4 or less. More specifically, examples thereof include a methyl group, an ethyl group, a n-propyl group, an isopropyl group, a n-butyl group, a tert-butyl group, a sec-butyl group, a hexyl group, and a cyclohexyl group. The alkyl group may have a halogen atom as a substituent. Among halogen atoms, a fluorine atom may be used.

In this specification, the aryl group is, for example, an aryl group having 5 to 60 carbon atoms. The number of carbon atoms may be 5 or more and 30 or less, or may be 5 or more and 20 or less. More specific examples thereof include a phenyl group, a naphthyl group, an indenyl group, a biphenyl group, a terphenyl group, a fluorenyl group, a phenanthryl group, a fluoranthenyl group, and triphenylenyl group.

In this specification, the heteroaryl group may be a heteroaryl group, for example, having 3 or more and 58 or less carbon atoms. The number of carbon atoms may be 5 or more and 28 or less, or may be 5 or more and 18 or less. A heteroatom used may be a nitrogen atom, a sulfur atom, or an oxygen atom. Further specific examples thereof include a pyridyl group, an oxazolyl group, an oxadiazolyl group, a thiazolyl group, a thiadiazolyl group, a carbazolyl group, an acridinyl group, a phenanthrolinyl group, an azaphenanthryl group, a dibenzofuranyl group, a dibenzothiophenyl group, an azatriphenyl group, and a diazatriphenyl group.

In this specification, the aryl group and heteroaryl group may each have a substituent. The substituent is a deuterium atom, a halogen atom, or an alkyl group. The halogen atom and the alkyl group are as described above.

In the general formula, the fused polycyclic structure A may have four or more rings. More specifically, the fused polycyclic structure A may be selected from the following structures: 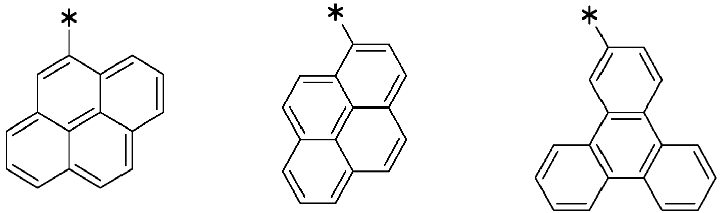 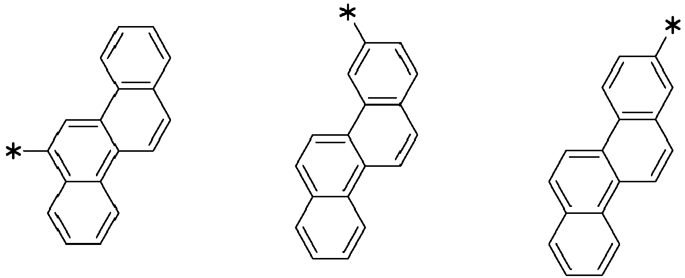 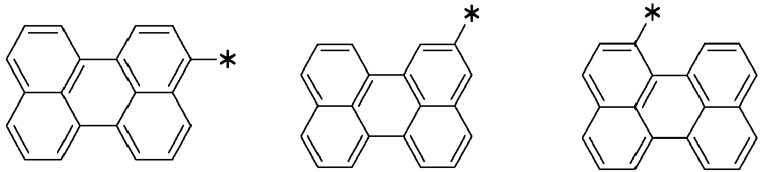 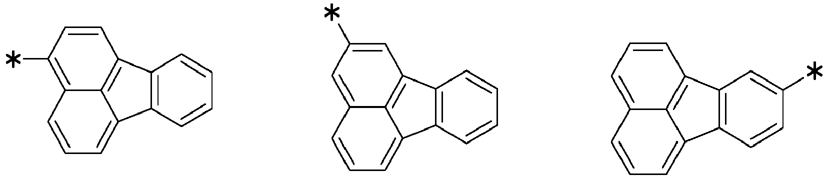 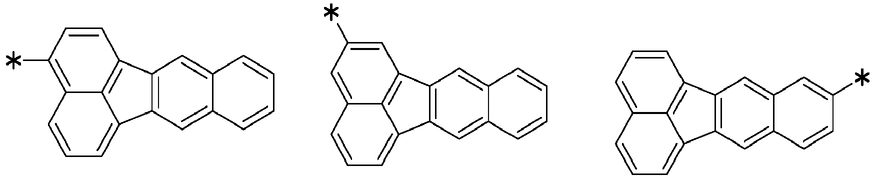 where each * represents a binding position.

The fused polycyclic structure A can be a perylene structure, a triphenylene structure, or a pyrene structure.

An element contained in the fused polycyclic structure A may be an element selected from the group consisting of a hydrogen atom, a deuterium atom, a nitrogen atom, an oxygen atom, and a sulfur atom.

In the general formula, the ring structure B is selected from a substituted or unsubstituted aryl group and a substituted or unsubstituted heteroaryl group. Examples of the aryl group and the heteroaryl group are the same as those of the aryl group and heteroaryl group in this specification.

The ring structure B may be an aryl group with four or less rings, or a heteroaryl group with four or less rings.

More specifically, the ring structure B can be selected from the group consisting of a phenyl group, a naphthyl group, and a fluorenyl group.

The element contained in the ring structure B may be selected from the group consisting of a hydrogen atom, a deuterium atom, a nitrogen atom, an oxygen atom, and a sulfur atom.

The compound represented by general formula [2] represents a multimer of A. n is an integer of 1 to 4. n can be 1. The fused polycyclic structures A have the same structure as an aryl group and may have different substituents.

The composition according to an embodiment of the present invention contains the organic compound AB represented by general formula [1] and the organic compound AA represented by general formula [2]. The proportion by weight of the organic compound AA may be more than 0.02% by weight and 50% or less by weight based on the total weight of the organic compound AB and the organic compound AA.

The proportion by weight of the organic compound AA may be more than 0.02% by weight and 30% or less by weight based on the total weight of the organic compound AB and the organic compound AA. The proportion by weight of the organic compound AA may be more than 0.02% by weight and 10% or less by weight based on the total weight of the organic compound AB and the organic compound AA.

The proportion by weight of the organic compound AA may be more than 0.1% by weight and 50% or less by weight based on the total weight of the organic compound AB and the organic compound AA. The proportion by weight of the organic compound AA may be more than 0.1% by weight and 30% or less by weight based on the total weight of the organic compound AB and the organic compound AA. The proportion by weight of the organic compound AA may be more than 0.1% by weight and 10% or less by weight based on the total weight of the organic compound AB and the organic compound AA.

### Action of Composition According to Embodiment of the Present Disclosure

When the composition of an embodiment of the present invention is used in an organic light-emitting device, the composition has the effect of extending the operating lifetime of the organic light-emitting device. To extend the operating lifetime of the organic light-emitting device, the deterioration of the organic compounds in the organic light-emitting device due to driving can be reduced. One way to extend the operating lifetime of the organic light-emitting device is to expand a zone where holes and electrons recombine in the light-emitting layer of the organic light-emitting device, thereby reducing the concentration of exciton generation. When exciton generation is concentrated, energy transfer from a molecule in another excited state may occur on the molecule in the excited state to result in a transition to a higher energy state. In a high-energy state, if the energy is more than the bond energy of a single-bond site in the molecular structure, the bond may be cleaved to generate a decomposition product. The decomposition product affects other molecules to cause a decrease in luminance. To avoid this, it is effective to disperse the generation of excitons.

The composition according to an embodiment of the present invention contains the organic compound AB and the organic compound AA having a molecular structure in which a partial structure of the organic compound AB is polymerized. When the compound is used in an organic light-emitting device, charge injection into a light-emitting layer is satisfactory, charge trapping properties are appropriately improved, and regions where excitons are generated can be dispersed.

The action of the composition in one embodiment of the present invention will be described below.

The use of the organic compound AB and the organic compound AA improves the charge trapping properties to improve the operating lifetime of the organic light-emitting device. The organic compound AB and the organic compound AA can be used in the same organic compound layer, or even in the same organic light-emitting layer.

The use of the organic compound AB and the organic compound AA makes it possible to adjust the charge mobility of the organic compound layer. When the organic compound AB and the organic compound AA are mixed, a charge trap level can be formed in the molecular orbital of the organic compound AB and the charge mobility can be reduced as compared with the case where only the organic compound AB is used.

In an organic compound layer composed of only a single organic compound, the charge mobility is uniquely determined. In contrast, when different organic compounds are mixed, a charge trap level can be formed to reduce the charge mobility. However, if two completely different types of organic compounds are used, for example, if an organic compound such as an organic compound XX having even a different partial structure from the organic compound AB is used, it is difficult to form an intended charge trap level because of a large difference in physical properties.

The composition according to an embodiment of the present invention contains the organic compound AB and the organic compound AA having a partial structure in common with the organic compound AB. The common partial structure can provide small differences in physical properties and high compatibility, thereby forming a trap level between the organic compound AB and the organic compound AA. Regarding the charge trap level, the molecular orbital energy levels can be different from each other within a certain range. The difference in molecular orbital energy level between the organic compound AB and the organic compound AA is preferably more than 0.00 eV and less than 0.30 eV, more preferably 0.15 eV or less. Here, it does not matter which of the molecular orbital levels of the organic compound AB and the organic compound AA is higher. For example, when the lowest unoccupied molecular orbital (LUMO) level of the organic compound AA is lower than the LUMO level of the organic compound AB, the organic compound AA easily serves as an electron trap. This is more noticeable when the proportion by weight of the organic compound AA is smaller than that of the organic compound AB.

When the highest occupied molecular orbital (HOMO) level of the organic compound AA is higher than the HOMO level of the organic compound AB, the organic compound AA easily serves as a hole trap. This is more noticeable when the proportion by weight of the organic compound AA is smaller than that of the organic compound AB.

As described above, the organic compound AA serves as a charge trap level and has the same partial structure as the organic compound AB, so that a stable organic layer can be formed.

### Bond of Organic Compound Contained in Composition According to Embodiment of the Present Disclosure

In the organic compound AA in the composition according to an embodiment of the present invention, all the freely rotatable single bonds are carbon-carbon bonds, and can be SP2 carbon-SP2 carbon bonds.

The composition according to one embodiment of the present invention has a configuration that can contribute to recombination and exciton generation on the organic compound AB and the organic compound AA. Energy is concentrated on the organic compound AB and the organic compound AA in an excited state. For this reason, the organic compound AB and the organic compound AA can have skeletons resistant to decomposition even in a high-energy excited state. The skeletons resistant to decomposition, specifically, indicate that all the freely rotatable single bonds are carbon-carbon bonds. The bonds can be SP2 carbon-SP2 carbon bonds.

As illustrated below with regard to examples of bond energy, when a carbonnitrogen bond or the like is provided as a bond between fused polycyclic skeletons, the bond is easily broken in a higher-order excitation energy state generated by a certain concentration of exciton generation.

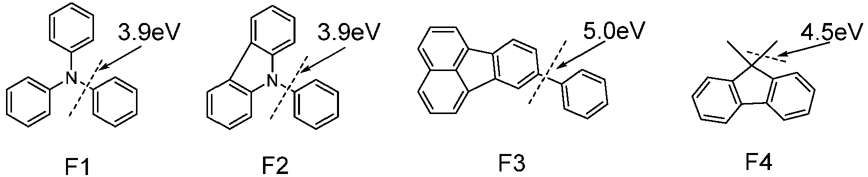

The bond energy is stable in order of F1, F2 (C-N) < F4 (SP3 carbon-SP3 carbon) < F3 (SP2 carbon-SP2 carbon). That is, a higher bond energy described is more stable.

### Film Stability of Organic Compound Contained in Composition According to Embodiment of the Present Disclosure

A composition according to an embodiment of the present invention contains the organic compound AB and the organic compound AA. The organic compound AB has the fused polycyclic structure A with two or more rings in its molecular structure and thus tends to have a high glass transition temperature. For this reason, the organic compound AB has high thermal stability and can maintain an amorphous state in a film form. In other words, even after a thin film of the organic compound AB is formed by vapor deposition, the organic compound AB is stable. This tendency is exhibited when the organic compound AB has a ring structure in which two or more rings are fused in its molecular structure. The tendency is exhibited more prominently in a ring structure in which four or more rings are fused.

Organic compounds for use in organic semiconductor devices, such as organic light-emitting devices, are highly purified by sublimation purification. The presence of the fused polycyclic structure A tends to increase the melting point of the organic compound. This allows the organic compound to remain in a solid state even in a high vacuum and to sublimate more easily. This tendency is exhibited more noticeably when a ring structure in which four or more rings are fused is contained. When the compound does not have a fused polycyclic structure, the compound tends to have a low melting point. Thus the compound is likely to be in a liquid state by heating in a high vacuum. The compound evaporated from a liquid state easily forms an azeotrope with an impurity and may contain an impurity in a film state. That is, the presence of the fused polycyclic structure A facilitates separation and purification to easily provide high purity.

### Organic Compound AA Represented by General Formula [2]

The organic compound AA has a structure in which n fused polycyclic structure(s) A is/are bonded. The organic compound AA can have a dimeric structure in which one fused polycyclic structure A is bonded. That is, n can be 1.

The design of the organic compound AA having a structure in which n fused polycyclic structure(s) A is/are bonded provides the following effects.

First, in a structure in which n fused polycyclic structure(s) A having a large molecular weight is/are bonded, a conformation that breaks the planarity is formed, and thus molecular stacking in which molecules overlap with each other can be reduced. A vapor-deposited film of the organic compound in which molecular stacking is reduced is less likely to crystallize and is likely to remain amorphous.

The organic compound having a high degree of amorphous nature, that is, good film properties, can be used in an organic light-emitting device. This is because the high degree of amorphous nature is less likely to lead to the generation of a crystal grain boundary and the formation of a trap level and a quencher due to microcrystallization even during the driving of the device, and thus results in the maintenance of good carrier transportability and highly efficient light-emitting characteristics. Thereby, the organic light-emitting device has excellent luminous efficiency and operating lifetime.

The lower symmetry in the molecular structure reduces the overlap between molecules, making it less likely to crystallize and more likely to maintain the amorphous state. In this case, the deposition of the organic compound AB and the organic compound AA in the same organic layer is more effective. The effect of steric hindrance is greater for a structure in which two or more fused aromatic rings where two or more rings are fused together are bonded together. The presence of the large fused aromatic ring composed of two or more rings in the space can provide the effect of steric hindrance. The effect is greater in a fused polycyclic compound with three or more rings, and even greater in a fused polycyclic compound with four or more rings.

Third, since both the organic compound AA and the organic compound AB have the same fused polycyclic structure A as a partial structure, both the compounds have high compatibility in the co-deposited film of the organic compound AA and the organic compound AB. The organic compound AA has n fused polycyclic structure(s) A and thus has a larger molecular weight. Accordingly, the co-deposited film of the organic compound AA and the organic compound AB is more effective in improving the thermal stability than the film of the organic compound AB alone.

As described above, the amorphous state can be easily maintained by increasing the molecular weight and breaking the symmetry. Thus, a stable amorphous state can be maintained during device operation, providing a long-life organic light-emitting device.

These effects are effectively provided by the fact that n fused polycyclic structures A are bonded. These effects can be more effectively provided by the organic compound AA in which two fused polycyclic structures A are bonded to each other. That is, n can be 1.

### Configuration of Composition

When the organic compounds in the composition according to an embodiment of the present invention further have the following configuration, they can be particularly used in an organic light-emitting device.

### (1) Structure of Fused Polycyclic Structure A

The fused polycyclic structure A is a fused polycyclic structure having a structure in which two or more five- or six-membered rings composed of an aromatic hydrocarbon composed of only a hydrocarbon or a heteroaromatic compound containing a hetero atom in its ring structure are fused. A fused structure of four or more aromatic rings can be used because the structure has a larger molecular weight and can easily form a stable structure having a smaller number of rotational sites as a partial structure.

While specific examples of the fused polycyclic structure A are illustrated below, the fused polycyclic structure A is not limited to these examples, 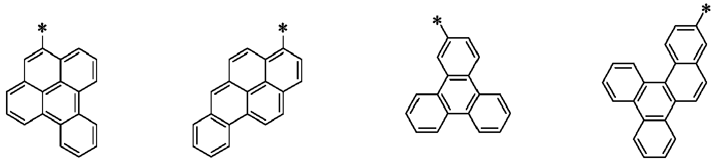 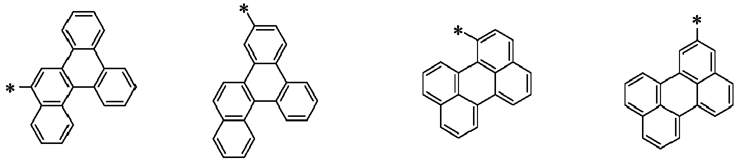 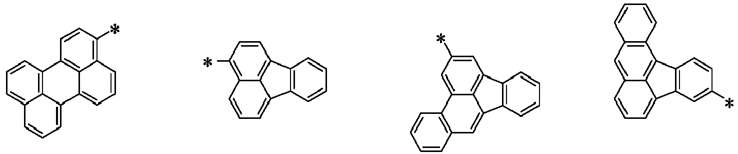 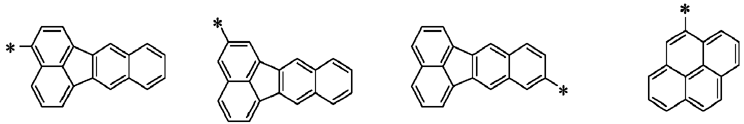 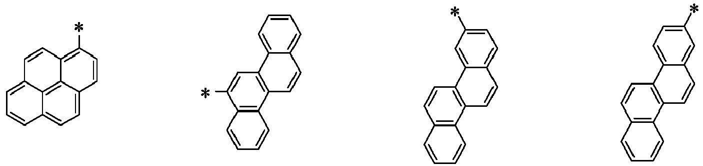 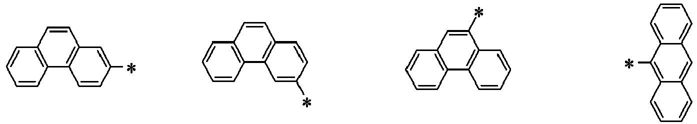 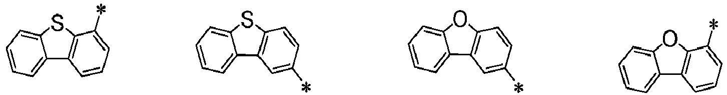 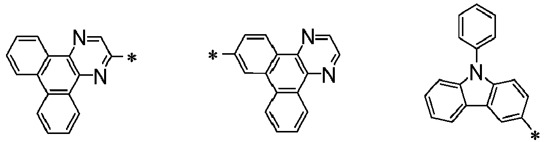 where each * represents a binding position.

The following structures can be used for the fused polycyclic structure A: 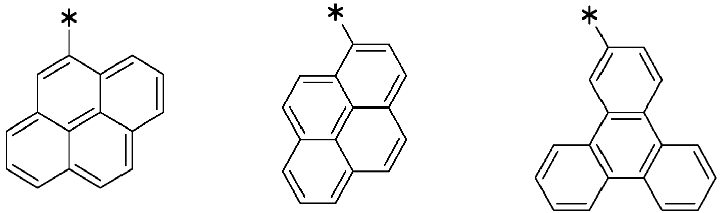 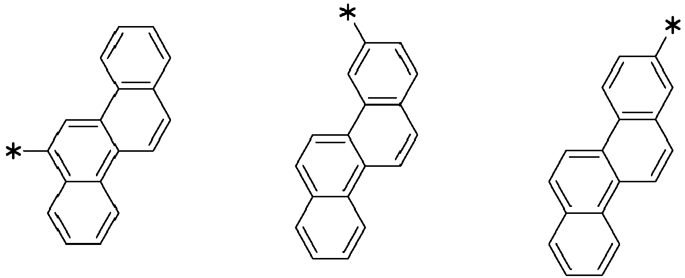 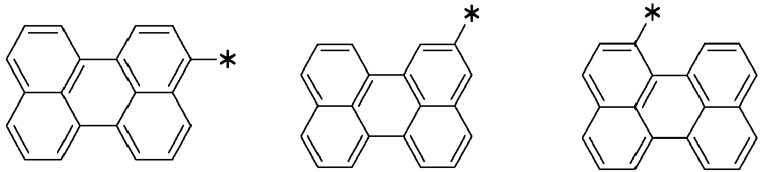 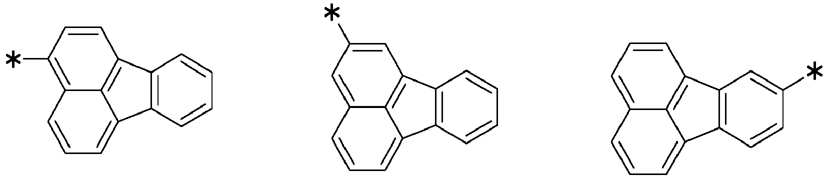 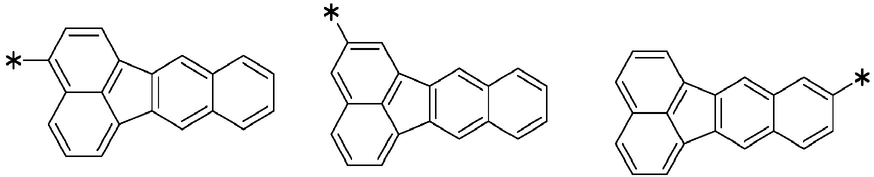 where each * represents a binding position.

### (2) Structure of Ring Structure B

In the organic compound AB, all the freely rotatable single bonds can be carbon-carbon bonds. The carbon-carbon bonds can be SP2 carbon-SP2 carbon bonds. An aromatic unit represented by the following general formula [3] can be contained.

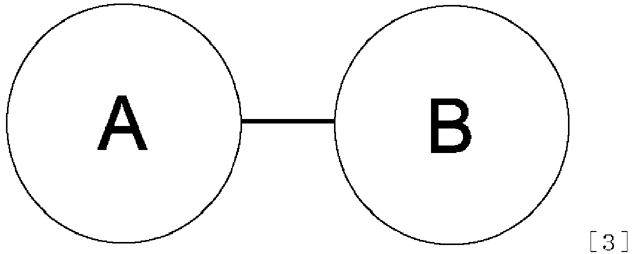

The ring structure B is a substituted or unsubstituted aryl group or a substituted or unsubstituted heteroaryl group, and may be fused.

The ring structure B may be a partial structure in which structures selected from the aryl group and the heteroaryl group are bonded to each other.

Examples of the aryl group and the heteroaryl group are the same as those of the aryl group and heteroaryl group in this specification.

### Specific Examples of Organic Compound AA

While specific examples of the organic compound AA according to an embodiment of the present invention are illustrated below, the present invention is not limited to these examples.

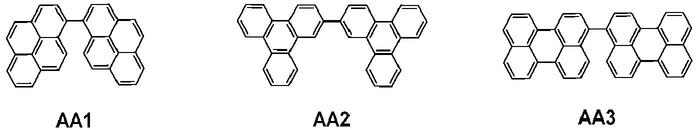

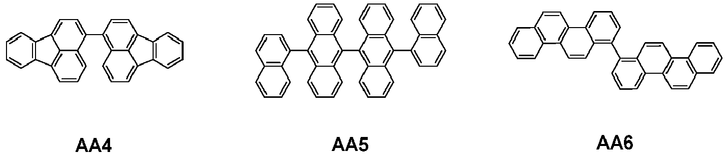

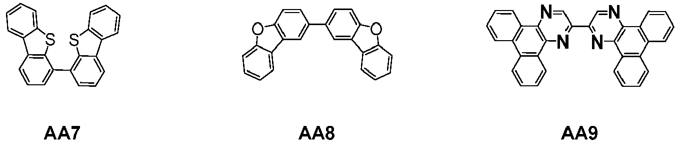

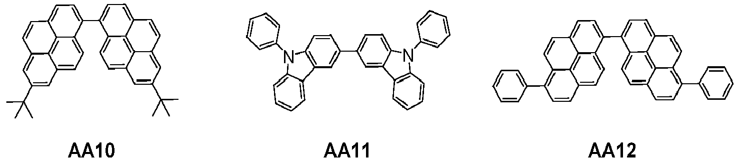

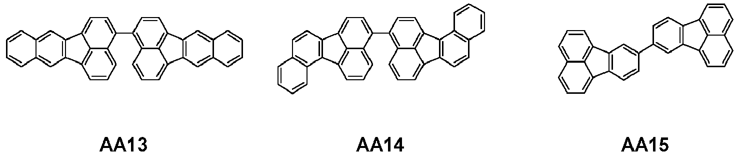

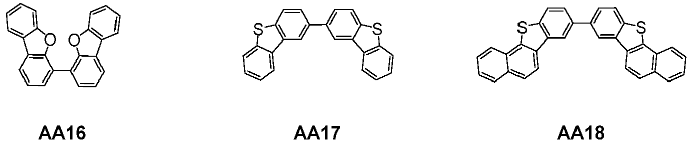

### Mode of Use of Composition

The organic compounds in the composition according to an embodiment of the present invention may be used in an organic compound layer of an organic light-emitting device under the following conditions.

(1) The organic compound AB can be used as a host material of the light-emitting layer. The proportion by weight of the host material in the light-emitting layer may be 50% or more by weight. When the organic compound AA mixed with the host material is used as a second host material, the organic compound AA is used in an amount of more than 0.02% by weight and 40% or less by weight, preferably 0.5% or more by weight and 30% or less by weight.

The organic compound AB in the composition according to an embodiment of the present invention is easily maintained in an amorphous state. Thus, the organic compound AB can be used as a host material in the light-emitting layer and can be used in an amount of about 50% or more by weight. The organic compound AB is not easily crystallized even when used in an amount of 99.9% by weight; thus, a function excellent in characteristics can be exhibited.

The organic compound AA may be used as the second host material from the viewpoint of improving the film properties of the light-emitting layer. When the organic compound AA is used as the second host material, the organic compound AA can be used in an amount of more than 0.02% by weight and less than 50% by weight. This is because the second host material having a dimeric structure of the fused polycyclic structure A can improve the amorphous nature even if the second host material is contained in the film in an amount of only 0.5% by weight.

When the light-emitting layer containing the composition according to an embodiment of the present invention contains the light-emitting compound, the organic compound AB, and the organic compound AA, the organic compound AB, the organic compound AA, and the light-emitting compound may be in decreasing order of energy gaps thereof. The energy gaps correspond to the lowest excited singlet energy (S 1) levels of the respective organic compounds, and can also be calculated from their HOMO and LUMO levels.

When the light-emitting layer containing the composition according to an embodiment of the present invention contains the light-emitting compound, the organic compound AB, and the organic compound AA, the organic compound AB, the organic compound AA, and the light-emitting compound may be in decreasing order of proportions by weight thereof. The organic compound AB is a host material, the organic compound AA is an assist material or a second host material, and the light-emitting compound is a dopant material.

The composition according to an embodiment of the present invention is not limited to one type. For example, a second composition containing the organic compound AB, the organic compound AA, an organic compound CD different from the organic compound AB, and an organic compound CC may be contained. Examples of the organic compound CD and the organic compound CC are the same as those of the organic compound AB and the organic compound AA, respectively. The organic compound CD is composed of a fused polycyclic structure C and a ring structure D, and is similar to the organic compound AB.

C-D [4]

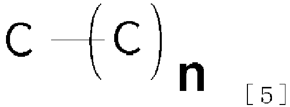

General formula [4] is the same as general formula [1], and general formula [5] is the same as general formula [2].

The second composition according to an embodiment of the present invention may be used in a light-emitting layer different from a light-emitting layer in which the composition is used.

That is, when the composition according to an embodiment of the present invention is used in a light-emitting layer, the second composition may be used in a second light-emitting layer different from the light-emitting layer. The light-emitting layer and the second light-emitting layer may be in contact with each other, or may be stacked with an additional organic compound layer interposed therebetween. The additional organic compound layer may be a charge generation layer described below.

This depends on the composition of the organic compounds in the composition according to an embodiment of the present invention. It is possible to provide an organic light-emitting device in which a compound is less likely to aggregate, a crystal grain boundary due to molecular aggregation is less likely to be generated even when the organic light-emitting device is driven, and which has excellent characteristics. Organic Light-Emitting Device

An organic light-emitting device according to an embodiment will be described below. The organic light-emitting device according to the present embodiment includes at least a first electrode, a second electrode, and an organic compound layer disposed between these electrodes.

One of the first electrode and the second electrode is an anode, and the other is a cathode. In the organic light-emitting device according to the present embodiment, the organic compound layer may be formed of a single layer or a laminate including multiple layers, as long as it includes a light-emitting layer. When the organic compound layer is formed of a laminate including multiple layers, the organic compound layer may include, in addition to the light-emitting layer, a hole injection layer, a hole transport layer, an electron-blocking layer, a hole-exciton-blocking layer, an electron transport layer, and an electron injection layer, for example. The light-emitting layer may be formed of a single layer or a laminate including multiple layers. In the case of providing multiple light-emitting layers, a charge generation layer may be disposed between the light-emitting layers. The charge generation layer may be composed of a compound having a LUMO level lower than the hole transport layer, and the LUMO level of the charge generation layer may be lower than the HOMO level of the hole transport layer. Here, the molecular orbital energy level of the organic compound layer may be the molecular orbital energy level of the organic compound having the largest proportion by weight in the organic compound layer.

Here, the HOMO level and the LUMO level are described as "higher" as they are closer to the vacuum level. The fact that LUMO level of the charge generation layer is lower than the HOMO level of the hole transport layer indicates that the LUMO level of the charge generation layer is closer to the vacuum level than the HOMO level of the hole transport layer.

In this specification, HOMO and LUMO can be calculated by molecular orbital calculation. As the molecular orbital calculation method, the density functional theory (DFT) may be used with the B3LYP functional and 6-3 1 G* as the basis function, for example. The molecular orbital calculation method was performed using Gaussian 09 (Gaussian 09, Revision C.01, M.J. Frisch, G.W. Trucks, H.B. Schlegel, G.E. Scuseria, M.A. Robb, J.R. Cheeseman, G. Scalmani, V. Barone, B. Mennucci, G.A. Petersson, H. Nakatsuji, M. Caricato, X. Li, H.P. Hratchian, A.F. Izmaylov, J. Bloino, G. Zheng, J.L. Sonnenberg, M. Hada, M. Ehara, K. Toyota, R. Fukuda, J. Hasegawa, M. Ishida, T. Nakajima, Y. Honda, O. Kitao, H. Nakai, T. Vreven, J.A. Montgomery, Jr., J.E. Peralta, F. Ogliaro, M. Bearpark, J.J. Heyd, E. Brothers, K.N. Kudin, V.N. Staroverov, T. Keith, R. Kobayashi, J. Normand, K. Raghavachari, A. Rendell, J.C. Burant, S.S. Iyengar, J. Tomasi, M. Cossi, N. Rega, J.M. Millam, M. Klene, J.E. Knox, J.B. Cross, V. Bakken, C. Adamo, J. Jaramillo, R. Gomperts, R.E. Stratmann, O. Yazyev, A.J. Austin, R. Cammi, C. Pomelli, J.W. Ochterski, R.L. Martin, K. Morokuma, V.G. Zakrzewski, G.A. Voth, P. Salvador, J.J. Dannenberg, S. Dapprich, A.D. Daniels, O. Farkas, J.B. Foresman, J.V. Ortiz, J. Cioslowski, and D.J. Fox, Gaussian, Inc., Wallingford CT, 2010).

The HOMO and LUMO levels in this specification can be calculated using an ionization potential and a band gap. A HOMO level can be estimated by measuring an ionization potential. The ionization potential can be measured by dissolving a target compound in a solvent, such as toluene, and performing measurement with a measurement apparatus, such as AC-3. The band gap can be measured by dissolving a target compound in a solvent, such as toluene, and then irradiating the resulting solution with excitation light. The band gap can be measured by measuring the absorption edge with the excitation light. Alternatively, the measurement can be performed by depositing a target compound using vapor deposition on a substrate, such as a glass substrate, and irradiating the resulting film with excitation light. The band gap can be measured by measuring the absorption edge of the absorption spectrum of the film when the film absorbs the excitation light.

The LUMO level can be calculated using the values of the band gap and the ionization potential. The LUMO level can be estimated by subtracting the value of the ionization potential from the value of the band gap.

The LUMO level can also be estimated from a reduction potential. For example, a one-electron reduction potential is estimated by cyclic voltammetry (CV) measurement. The CV measurement can be performed, for example, in a 0.1 M solution of tetrabutylammonium perchlorate in DMF using Ag/Ag+ as a reference electrode, Pt as a counter electrode, and glassy carbon as a working electrode. The LUMO level can be estimated by adding -4.8 eV to the difference between the reduction potential of the obtained compound and the reduction potential of ferrocene.

In the organic light-emitting device according to an embodiment of the present invention, when the organic compound according to the present embodiment is contained in a light-emitting layer, the light-emitting layer may be composed only of the organic compound according to the present embodiment or may be composed of an organometallic complex according to the present embodiment and another compound. When the light-emitting layer is composed of the organometallic complex according to the present embodiment and another compound, the organic compound according to the present embodiment may be used as a host or a guest of the light-emitting layer. The organic compound according to the present embodiment may be used as an assist material that can be contained in the light-emitting layer. The term "host" used here refers to a compound having the highest proportion by mass in compounds contained in the light-emitting layer. The term "guest" refers to a compound that has a lower proportion by mass than the host in the compounds contained in the light-emitting layer and that is responsible for main light emission. The term "assist material" refers to a compound that has a lower proportion by mass than the host in the compounds contained in the light-emitting layer and that assists the light emission of the guest. The assist material is also referred to as a "second host". The host material may be referred to as the "first compound". The assist material may be referred to as a "second compound".

When the organic compound according to an embodiment of the present invention is used as a guest in the light-emitting layer, the concentration of the guest is preferably 0.01% or more by mass and 20% or less by mass, more preferably 0.1% or more by mass and 10% or less by mass, based on the entire light-emitting layer. The guest is also referred to as a "dopant".

The inventors have conducted various studies and have found that when the organic compound according to the present embodiment is used as a host or guest of a light-emitting layer, especially as a guest of a light-emitting layer, a device that emits light with high efficiency and high luminance and that is extremely durable can be provided. This light-emitting layer can be formed of a single layer or multiple layers and can also contain a light-emitting material having another emission color in order to conduct the color mixture of the red emission color of the present embodiment and another emission color. The term "multiple layers" refers to a state in which a light-emitting layer and another light-emitting layer are stacked. In this case, the emission color of the organic light-emitting device is not limited to red. More specifically, the emission color may be white or an intermediate color. In the case of white, another light-emitting layer emits light of a color other than red, that is, blue or green. A filmforming method is vapor deposition or coating. Details will be described in examples below.

The organometallic complex according to the present embodiment can be used as a component material of an organic compound layer other than the light-emitting layer included in the organic light-emitting device according to the embodiment. Specifically, the organometallic complex may be used as a component material of the electron transport layer, the electron injection layer, the hole transport layer, the hole injection layer, the hole-blocking layer, and so forth. In this case, the emission color of the organic light-emitting device is not limited to red. More specifically, the emission color may be white or intermediate color.

For example, a hole injection compound, a hole transport compound, a compound to be used as a host, a light-emitting compound, an electron injection compound, or an electron transport compound, which is known and has a low or high molecular weight, can be used together with the organic compound according to the present embodiment, as needed. Examples of these compounds are illustrated below.

As a hole injection-transport material, a material having a high hole mobility can be used so as to facilitate the injection of holes from the anode and to transport the injected holes to the light-emitting layer. To reduce a deterioration in film quality, such as crystallization, in the organic light-emitting device, a material having a high glass transition temperature can be used. Examples of a low- or high-molecular-weight material having the ability to inject and transport holes include triarylamine derivatives, aryl carbazole derivatives, phenylenediamine derivatives, stilbene derivatives, phthalocyanine derivatives, porphyrin derivatives, poly(vinyl carbazole), polythiophene, and other conductive polymers. Moreover, the hole injection-transport material can also be used for the electron-blocking layer. Non-limiting specific examples of a compound used as the hole injection-transport material will be illustrated below.

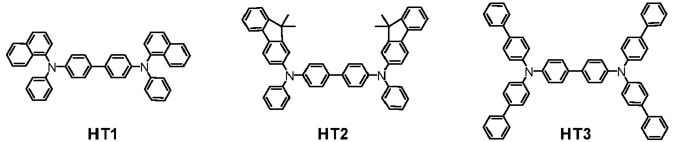

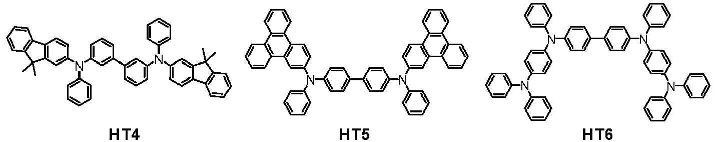

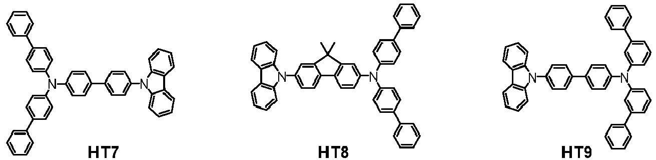

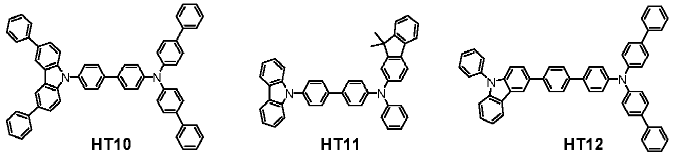

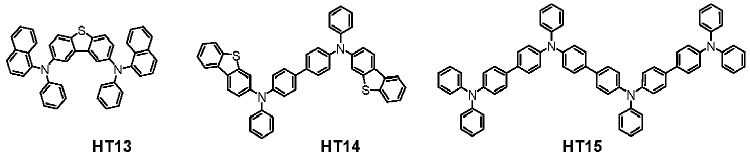

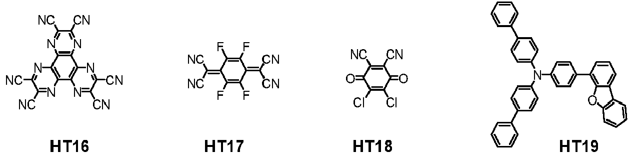

Examples of a light-emitting material mainly associated with a light-emitting function include, in addition to the organometallic complex related to the compounds in the light-emitting layer according to an embodiment of the present invention, fused-ring compounds, such as fluorene derivatives, naphthalene derivatives, pyrene derivatives, perylene derivatives, tetracene derivatives, anthracene compounds, and rubrene, quinacridone derivatives, coumarin derivatives, stilbene derivatives, organoaluminum complexes, such as tris(8-quinolinolato)aluminum, iridium complexes, platinum complexes, rhenium complexes, copper complexes, europium complexes, ruthenium complexes, and polymer derivatives, such as poly(phenylene vinylene) derivatives, polyfluorene derivatives, and polyphenylene derivatives. Non-limiting examples of a compound used as a light-emitting material are specifically described below.

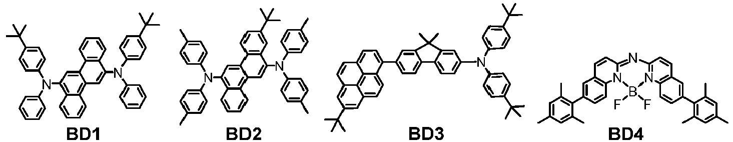

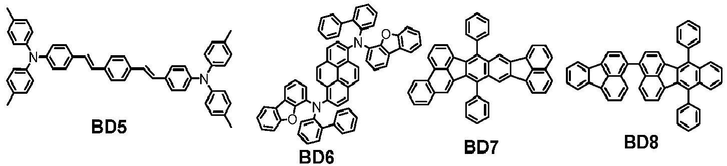

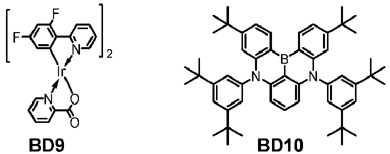

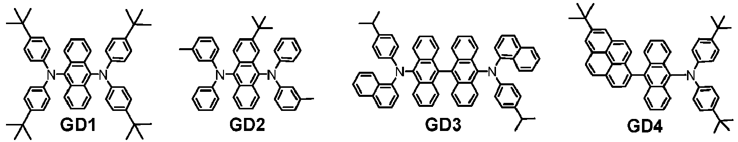

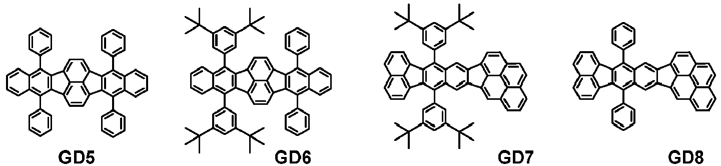

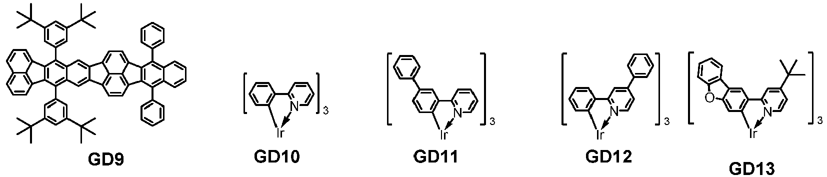

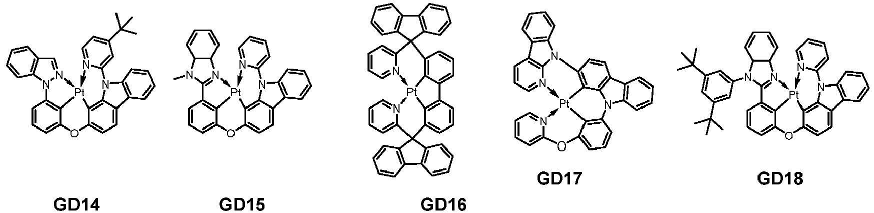

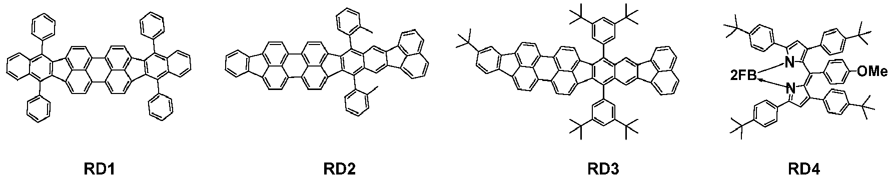

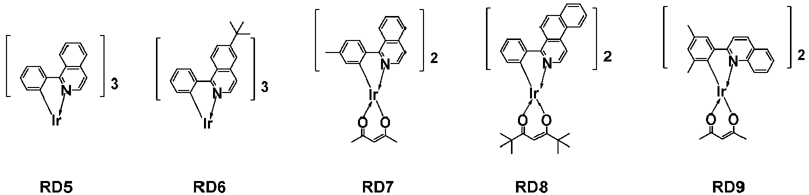

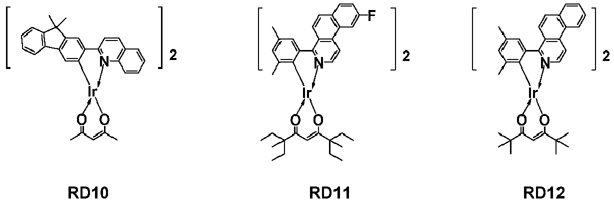

As a light-emitting layer host or a light-emission assist material contained in the light-emitting layer, a compound other than the organic compound of the present embodiment may be contained as a third component. Examples of the third component include aromatic hydrocarbon compounds and derivatives thereof, carbazole derivatives, azine derivatives, xanthone derivatives, dibenzofuran derivatives, dibenzothiophene derivatives, organoaluminum complexes such as tris(8-quinolinolato)aluminum, and organoberyllium complexes.

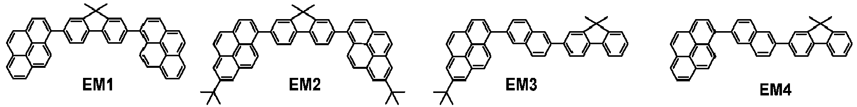

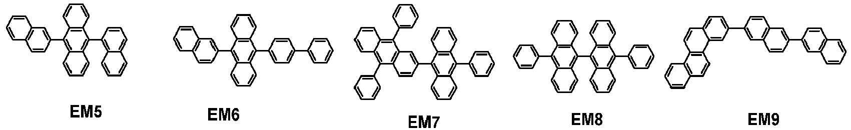

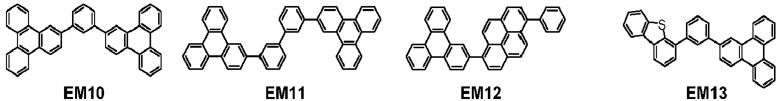

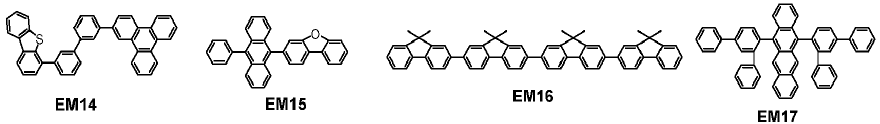

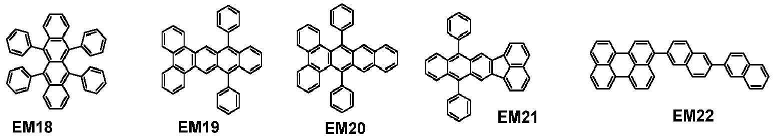

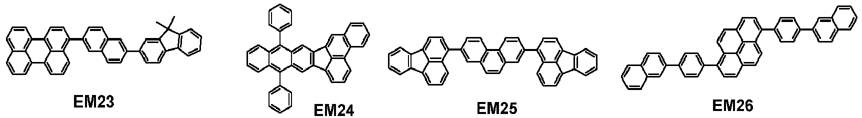

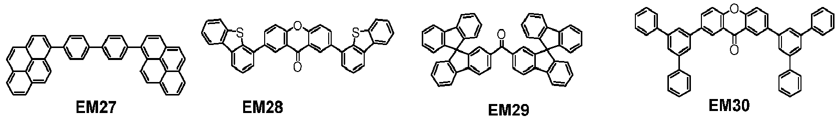

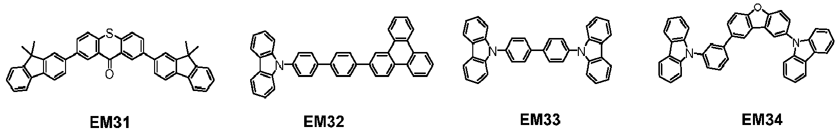

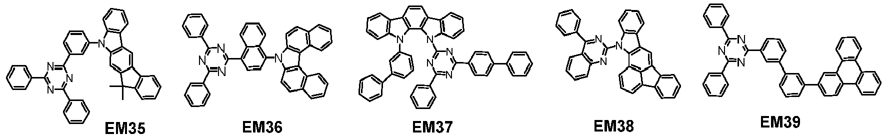

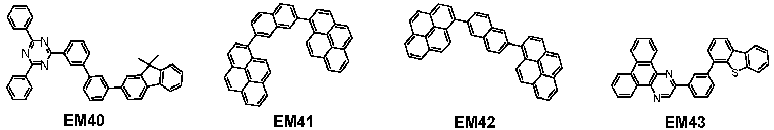

The electron transport material can be freely-selected from materials capable of transporting electrons injected from the cathode to the light-emitting layer and is selected in consideration of, for example, the balance with the hole mobility of the hole transport material. Examples of a material having the ability to transport electrons include oxadiazole derivatives, oxazole derivatives, pyrazine derivatives, triazole derivatives, triazine derivatives, quinoline derivatives, quinoxaline derivatives, phenanthroline derivatives, organoaluminum complexes, and fused-ring compounds, such as fluorene derivatives, naphthalene derivatives, chrysene derivatives, and anthracene derivatives. The electron transport materials can be used for the hole-blocking layer. Non-limiting examples of a compound used as the electron transport material will be specifically described below.

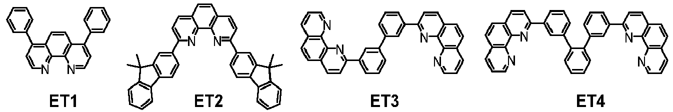

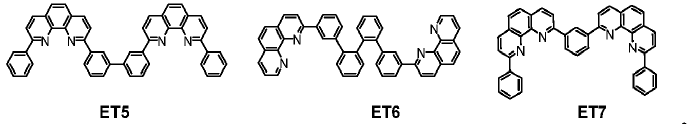

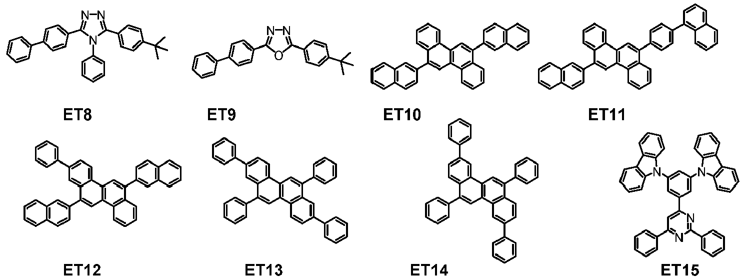

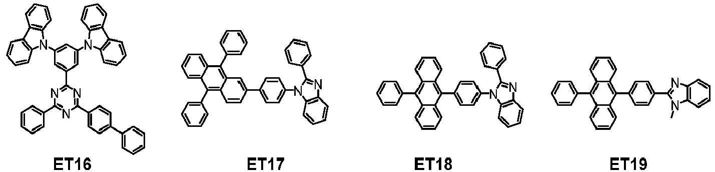

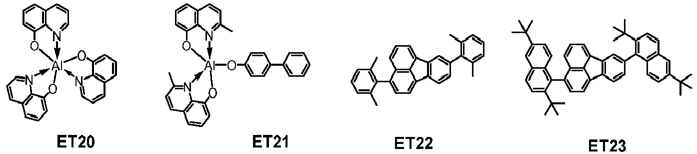

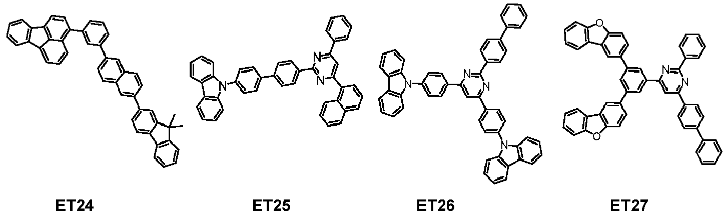

An electron injection material can be freely-selected from materials into which electrons can be easily injected from the cathode and is selected in consideration of, for example, the balance with the hole injection properties. As the organic compound, n-type dopants and reducing dopants are also included. Examples thereof include alkali metal-containing compounds such as lithium fluoride, lithium complexes such as lithium quinolinolate, benzimidazolidene derivatives, imidazolidene derivatives, fulvalene derivatives, and acridine derivatives. It can also be used in combination with the above-mentioned electron transport material.

### Configuration of Organic Light-Emitting Device

The organic light-emitting device includes an insulating layer, a first electrode, an organic compound layer, a second electrode over a substrate. A protective layer, a color filter, a microlens, and so forth may be disposed over the cathode.

In the case of disposing the color filter, a planarization layer may be disposed between the protective layer and the color filter. The planarization layer can be composed of, for example, an acrylic resin. The same applies when a planarization layer is provided between the color filter and the microlens.

### Substrate

Examples of the substrate include silicon wafers, quartz substrates, glass substrates, resin substrates, and metal substrates. The substrate may include a switching element, such as a transistor, a line, and an insulating layer thereon. Any material can be used for the insulating layer as long as a contact hole can be formed in such a manner that a line can be coupled to the first electrode and as long as insulation with a nonconnected line can be ensured. For example, a resin, such as polyimide, silicon oxide, or silicon nitride, can be used.

### Electrode

A pair of electrodes can be used. The pair of electrodes may be an anode and a cathode.

In the case where an electric field is applied in the direction in which the organic light-emitting device emits light, an electrode having a higher potential is the anode, and the other is the cathode. It can also be said that the electrode that supplies holes to the light-emitting layer is the anode and that the electrode that supplies electrons is the cathode.

As the component material of the anode, a material having a work function as high as possible can be used. Examples of the material that can be used include elemental metals, such as gold, platinum, silver, copper, nickel, palladium, cobalt, selenium, vanadium, and tungsten, mixtures thereof, alloys of combinations thereof, and metal oxides, such as tin oxide, zinc oxide, indium oxide, indium-tin oxide (ITO), and indium-zinc oxide. Additionally, conductive polymers, such as polyaniline, polypyrrole, and polythiophene, can be used.

These electrode materials may be used alone or in combination of two or more. The anode may be formed of a single layer or multiple layers.

When the anode is used as a reflective electrode, for example, chromium, aluminum, silver, titanium, tungsten, molybdenum, an alloy thereof, or a stack thereof may be used. These materials can also be used to act as a reflective film that does not have the role of an electrode. When the anode is used as a transparent electrode, a transparent conductive oxide layer composed of, for example, indium-tin oxide (ITO) or indium-zinc oxide may be used; however, the anode is not limited thereto.

The electrode can be formed by photolithography.

As the component material of the cathode, a material having a lower work function can be used. Examples thereof include elemental metals such as alkali metals, e.g., lithium, alkaline-earth metals, e.g., calcium, aluminum, titanium, manganese, silver, lead, and chromium, and mixtures thereof. Alloys of combinations of these elemental metals can also be used. For example, magnesium-silver, aluminumlithium, aluminum-magnesium, silver-copper, and zinc-silver can be used. Metal oxides, such as indium-tin oxide (ITO), can also be used. These electrode materials may be used alone or in combination of two or more. The cathode may have a singlelayer structure or a multilayer structure. In particular, silver can be used. To reduce the aggregation of silver, a silver alloy can be used. Any alloy ratio may be used as long as the aggregation of silver can be reduced. The ratio of silver to another metal may be, for example, 1:1 or 3: 1.

A top emission device may be provided using the cathode formed of a conductive oxide layer composed of, for example, ITO. A bottom emission device may be provided using the cathode formed of a reflective electrode composed of, for example, aluminum (Al). Any type of cathode may be used. Any method for forming the cathode may be employed. For example, a direct-current or alternating-current sputtering technique can be employed because good film coverage is obtained and thus the resistance is easily reduced.

### Pixel Separation Layer

The pixel separation layer is formed of a silicon nitride (SiN) film, a silicon oxynitride (SiON) film, or a silicon oxide (SiO) film formed by a chemical vapor deposition (CVD) method.

To increase the resistance in the in-plane direction of the organic compound layer, the organic compound layer, in particular, the hole transport layer, can be formed so as to have a small thickness at the side wall of the pixel separation layer. Specifically, the organic compound layer can be formed so as to have a small thickness at the side wall of the pixel separation layer by increasing the taper angle of the side wall of the pixel separation layer or the thickness of the pixel separation layer to increase vignetting at the time of vapor deposition.

The taper angle of the side wall of the pixel separation layer and the thickness of the pixel separation layer can be adjusted to such an extent that a void is not formed in the protective layer formed over the pixel separation layer. Voids are not formed in the protective layer; thus, the occurrence of defects in the protective layer can be reduced. Since the occurrence of defects in the protective layer is reduced, it is possible to reduce reliability deterioration, such as the formation of dark spots and the occurrence of poor conduction of the second electrode.

According to the embodiment, even if the taper angle of the side wall of the pixel separation layer is not steep, it is possible to effectively inhibit charge leakage to an adjacent pixel. As a result of this study, it has been found that a sufficient reduction can be achieved when the taper angle is 60° or more and 90° or less. The pixel separation layer can have a thickness of 10 nm or more and 150 nm or less. The same effect can be provided even if the pixel electrode does not include the pixel separation layer. However, in this case, the thickness of the pixel electrode can be less than or equal to the half of the thickness of the organic layer, or the pixel electrode can have a forward tapered end portion of less than 60°, because the short circuit of the organic light-emitting device can be reduced.

### Organic Compound Layer

The organic compound layer may be formed of a single layer or multiple layers. When multiple layers are present, they may be referred to as a hole injection layer, a hole transport layer, an electron-blocking layer, a light-emitting layer, a hole-blocking layer, an electron transport layer, or an electron injection layer in accordance with their functions. The organic compound layer is mainly composed of an organic compound, and may contain inorganic atoms and an inorganic compound. For example, the organic compound layer may contain, for example, copper, lithium, magnesium, aluminum, iridium, platinum, molybdenum, or zinc. The organic compound layer may be disposed between the first electrode and the second electrode, and may be disposed in contact with the first electrode and the second electrode.

### Protective Layer

A protective layer may be disposed on the second electrode. For example, a glass member provided with a moisture absorbent can be bonded to the second electrode to reduce the entry of, for example, water into the organic compound layer, thereby reducing the occurrence of display defects. In another embodiment, a passivation film composed of, for example, silicon nitride may be disposed on the cathode to reduce the entry of, for example, water into the organic compound layer. For example, after the formation of the cathode, the substrate may be transported to another chamber without breaking the vacuum, and a silicon nitride film having a thickness of 2 µm may be formed by a chemical vapor deposition (CVD) method to provide a protective layer. After the film deposition by the CVD method, a protective layer may be formed by an atomic layer deposition (ALD) method. Non-limiting examples of the material of the layer formed by the ALD method may include silicon nitride, silicon oxide, and aluminum oxide. Silicon nitride may be deposited by the CVD method on the layer formed by the ALD method. The film formed by the ALD method may have a smaller film thickness than the film formed by the CVD method. Specifically, the thickness may be 50% or less, even 10% or less.

### Color Filter

A color filter may be disposed on the protective layer. For example, a color filter may be disposed on another substrate in consideration of the size of the organic light-emitting device and bonded to the substrate provided with the organic light-emitting device. A color filter may be formed by patterning on the protective layer using photolithography. The color filter may be composed of a polymer.

### Planarization Layer

A planarization layer may be disposed between the color filter and the protective layer. The planarization layer is provided for the purpose of reducing the unevenness of the layer underneath. The planarization layer may be referred to as a "material resin layer" without limiting its purpose. The planarization layer may be composed of an organic compound. A low- or high-molecular-weight organic compound may be used. A high-molecular-weight organic compound can be used.

The planarization layers may be disposed above and below (or on) the color filter and may be composed of the same or different component materials. Specific examples thereof include poly(vinyl carbazole) resins, polycarbonate resins, polyester resins, acrylonitrile butadiene styrene (ABS) resins, acrylic resins, polyimide resins, phenolic resins, epoxy resins, silicone resins, and urea resins.

### Microlens

The organic light-emitting apparatus may include an optical member, such as a microlens, on the outgoing light side. The microlens can be composed of, for example, an acrylic resin or an epoxy resin. The microlens may be used to increase the amount of light emitted from the organic light-emitting apparatus and to control the direction of the light emitted. The microlens may have a hemispherical shape. In the case of a hemispherical shape, among tangents to the hemisphere, there is a tangent parallel to the insulating layer. The point of contact of the tangent with the hemisphere is the vertex of the microlens. The vertex of the microlens can be determined in the same way for any cross-sectional view. That is, among the tangents to the semicircle of the microlens in the cross-sectional view, there is a tangent parallel to the insulating layer, and the point of contact of the tangent with the semicircle is the vertex of the microlens.

The midpoint of the microlens can be defined. In the cross section of the microlens, when a segment is hypothetically drawn from the point where an arc shape ends to the point where another arc shape ends, the midpoint of the segment can be referred to as the midpoint of the microlens. The cross section to determine the vertex and midpoint may be a cross section perpendicular to the insulating layer.

The microlens has a first surface having a convex portion and a second surface opposite to the first surface. The second surface can be disposed closer to the functional layer than the first surface. To form such a configuration, it is necessary to form a microlens on the light-emitting apparatus. When the functional layer is an organic layer, a high-temperature process can be avoided in the production process. When the second surface is closer to the functional layer than the first surface, the glass transition temperature of each organic compound constituting the organic layer is preferably 100°C or higher, more preferably 130°C or higher.

### Opposite Substrate

An opposite substrate may be disposed on the planarization layer. The opposite substrate is disposed at a position corresponding to the substrate described above and thus is called an opposite substrate. The opposite substrate may be composed of the same material as the substrate described above. When the above-described substrate is referred to as a first substrate, the opposite substrate may be referred to as a second substrate.

### Organic Layer

The organic compound layer, such as the hole injection layer, the hole transport layer, the electron-blocking layer, the light-emitting layer, the hole-blocking layer, the electron transport layer, or the electron injection layer, included in the organic light-emitting device according to an embodiment of the present invention is formed by a method described below.

For the organic compound layer included in the organic light-emitting device according to an embodiment of the present invention, a dry process, such as a vacuum evaporation method, an ionized evaporation method, sputtering, or plasma, may be employed. Alternatively, instead of the dry process, it is also possible to employ a wet process in which a material is dissolved in an appropriate solvent and then a film is formed by a known coating method, such as spin coating, dipping, a casting method, a Langmuir-Blodgett (LB) technique, or an inkjet method.

When the layer is formed by, for example, the vacuum evaporation method or the solution coating method, crystallization and so forth are less likely to occur, and good stability with time is obtained. In the case of forming a film by the coating method, the film may be formed in combination with an appropriate binder resin.

Non-limiting examples of the binder resin include poly(vinyl carbazole) resins, polycarbonate resins, polyester resins, ABS resins, acrylic resins, polyimide resins, phenolic resins, epoxy resins, silicone resins, and urea resins.

These binder resins may be used alone as a homopolymer or copolymer or in combination as a mixture of two or more. Furthermore, additives, such as a known plasticizer, antioxidant, and ultraviolet absorber, may be used, as needed.

### Pixel Circuit

The light-emitting apparatus may include pixel circuits coupled to the light-emitting devices. Each of the pixel circuits may be of an active matrix type, which independently controls the emission of first and second light-emitting devices. The active matrix type circuit may be voltage programming or current programming. A driving circuit includes the pixel circuit for each pixel. The pixel circuit may include a light-emitting device, a transistor to control the luminance of the light-emitting device, a transistor to control the timing of the light emission, a capacitor to retain the gate voltage of the transistor to control the luminance, and a transistor to connect to GND without using the light-emitting device.

The light-emitting apparatus includes a display area and a peripheral area disposed around the display area. The display area includes a pixel circuit, and the peripheral area includes a display control circuit. The mobility of a transistor contained in the pixel circuit may be lower than the mobility of a transistor contained in the display control circuit.

The slope of the current-voltage characteristics of the transistor contained in the pixel circuit may be smaller than the slope of the current-voltage characteristic of the transistor contained in the display control circuit. The slope of the current-voltage characteristics can be measured by what is called Vg-Ig characteristics.

The transistor contained in the pixel circuit is a transistor coupled to a light-emitting device, such as a first light-emitting device.

### Pixel

The organic light-emitting apparatus includes multiple pixels. Each pixel includes subpixels configured to emit colors different from each other. The subpixels may have respective red, green, and blue (RGB) emission colors.

Light emerges from a region of the pixel, also called a pixel aperture. This region may also be referred to as a first region.

The pixel aperture may be 15 µm or less, and may be 5 µm or more. More specifically, the pixel aperture may be, for example, 11 µm, 9.5 µm, 7.4 µm, or 6.4 µm.

The distance between subpixels may be 10 µm. Specifically, the distance may be 8 µm, 7.4 µm, or 6.4 µm.

The pixels may be arranged in a known configuration in plan view. For example, a stripe pattern, a delta pattern, a Pen Tile matrix pattern, or the Bayer pattern may be used. The shape of each subpixel in plan view may be any known shape. Examples of the shape of the subpixel include quadrilaterals, such as rectangles and rhombi, and hexagons. Of course, if the shape is close to a rectangle, rather than an exact shape, it is included in the rectangle. The shape of the subpixel and the pixel arrangement can be used in combination.

### Application of Organic Light-Emitting Device According to Embodiment of the Present Disclosure

The organic light-emitting device according to an embodiment of the present invention can be used as a component member of a display apparatus or lighting apparatus. Other applications include exposure light sources for electrophotographic image-forming apparatuses, backlights for liquid crystal display apparatuses, and light-emitting apparatuses including white-light sources and color filters.

The display apparatus may be an image information-processing apparatus including an image input unit that receives image information from an area or linear CCD sensor, a memory card, or any other source, an information-processing unit that processes the input information, and a display unit that displays the input image.

The display unit of an image pickup apparatus or an inkjet printer may have a touch panel function. The driving mode of the touch panel function may be, but is not particularly limited to, an infrared mode, an electrostatic capacitance mode, a resistive film mode, or an electromagnetic inductive mode. The display apparatus may also be used for a display unit of a multifunction printer.

The following describes a display apparatus according to the present embodiment with reference to the attached drawings.

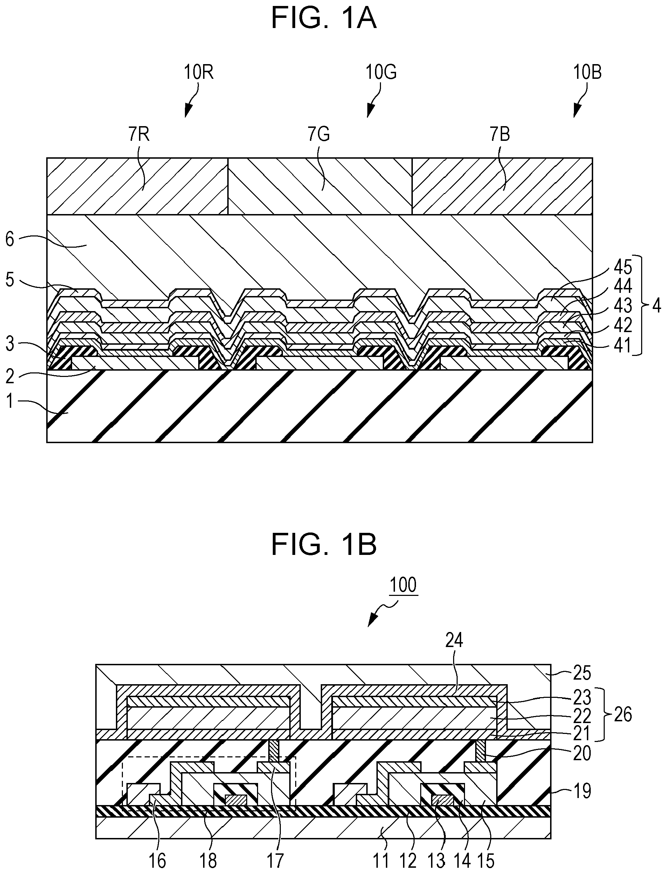

Figs. 1A and 1B are each a schematic cross-sectional view of an example of a display apparatus including organic light-emitting devices and transistors coupled to the respective organic light-emitting devices. Each of the transistors is an example of an active element. The transistors may be thin-film transistors (TFTs).

Fig. 1A is an example of pixels that are components of the display apparatus according to the present embodiment. Each of the pixels includes subpixels 10. The subpixels are divided into 10R, 10G, and 10B according to their light emission. The emission colors may be distinguished by the wavelength of light emitted from the light-emitting layer. Light emitted from the subpixels may be selectively transmitted or color-converted with, for example, a color filter. Each subpixels includes a reflective electrode 2 serving as a first electrode, an insulating layer 3 covering the edge of the reflective electrode 2, an organic compound layer 4 covering the first electrode and the insulating layer, a transparent electrode 5, a protective layer 6, and a color filter 7, over an interlayer insulating layer 1.

The transistors and capacitive elements may be disposed under or in the interlayer insulating layer 1.

Each transistor may be electrically coupled to a corresponding one of the first electrodes through a contact hole (not illustrated).

The insulating layer 3 is also called a bank or pixel separation film. The insulating layer covers the edge of each first electrode and surrounds the first electrode. Portions that are not covered with the insulating layer are in contact with the organic compound layer 4 and serve as light-emitting regions.

The organic compound layer 4 includes a hole injection layer 41, a hole transport layer 42, a first light-emitting layer 43, a second light-emitting layer 44, and an electron transport layer 45.

The second electrode 5 may be a transparent electrode, a reflective electrode, or a semi-transparent electrode.

The protective layer 6 reduces the penetration of moisture into the organic compound layer. Although the protective layer is illustrated as a single layer, the protective layer may include multiple layers, and each layer may be an inorganic compound layer or an organic compound layer.

The color filter 7 is separated into 7R, 7G, and 7B according to its color. The color filter may be disposed on a planarizing film (not illustrated). A resin protective layer (not illustrated) may be disposed on the color filter. The color filter may be disposed on the protective layer 6. Alternatively, the color filter may be disposed on an opposite substrate, such as a glass substrate, and then bonded.

A display apparatus 100 illustrated in Fig. 1B includes organic light-emitting devices 26 and TFTs 18 as an example of transistors. A substrate 11 composed of a material, such as glass or silicon, is provided, and an insulating layer 12 is disposed thereon. Active elements 18, such as the TFTs, are disposed on the insulating layer. The gate electrode 13, the gate insulating film 14, and the semiconductor layer 15 of each of the active elements are disposed thereon. Each TFT 18 further includes a semiconductor layer 15, a drain electrode 16, and a source electrode 17. The TFTs 18 are overlaid with an insulating film 19. Anode 21 included in the organic light-emitting devices 26 is coupled to the source electrodes 17 through contact holes 20 provided in the insulating film.

The mode of electrical connection between the electrodes (anode and cathode) included in each organic light-emitting device 26 and the electrodes (source electrode and drain electrode) included in a corresponding one of the TFTs is not limited to the mode illustrated in Fig. 1B. That is, it is sufficient that any one of the anode and the cathode is electrically coupled to any one of the source electrode and the drain electrode of the TFT. The term "TFT" refers to a thin-film transistor.

In the display apparatus 100 illustrated in Fig. 1B, although each organic compound layer 22 is illustrated as a single layer, the organic compound layer 22 may include multiple layers. To reduce the deterioration of the organic light-emitting devices, a first protective layer 24 and a second protective layer 25 are disposed on the cathodes 23.

In the display apparatus 100 illustrated in Fig. 1B, although the transistors are used as switching elements, other switching elements may be used instead.

The transistors used in the display apparatus 100 illustrated in Fig. 1B are not limited to transistors using a single-crystal silicon wafer, but may also be thin-film transistors including active layers on the insulating surface of a substrate. Examples of the material of the active layers include single-crystal silicon, non-single-crystal silicon, such as amorphous silicon and microcrystalline silicon; and non-single-crystal oxide semiconductors, such as indium zinc oxide and indium gallium zinc oxide. Thin-film transistors are also called TFT elements.

The transistors in the display apparatus 100 illustrated in Fig. 1B may be formed in the substrate, such as a Si substrate. The expression "formed in the substrate" indicates that the transistors are produced by processing the substrate, such as a Si substrate. In the case where the transistors are formed in the substrate, the substrate and the transistors can be deemed to be integrally formed.

In the organic light-emitting device according to the present embodiment, the luminance is controlled by the TFT devices, which are an example of switching elements; thus, an image can be displayed at respective luminance levels by arranging multiple organic light-emitting devices in the plane. The switching devices according to the present embodiment are not limited to the TFT devices and may be low-temperature polysilicon transistors or active-matrix drivers formed on a substrate such as a Si substrate. The expression "on a substrate" can also be said to be "in the substrate". Whether transistors are formed in the substrate or TFT devices are used is selected in accordance with the size of a display unit. For example, in the case where the display unit has a size of about 0.5 inches, organic light-emitting devices can be disposed on a Si substrate.

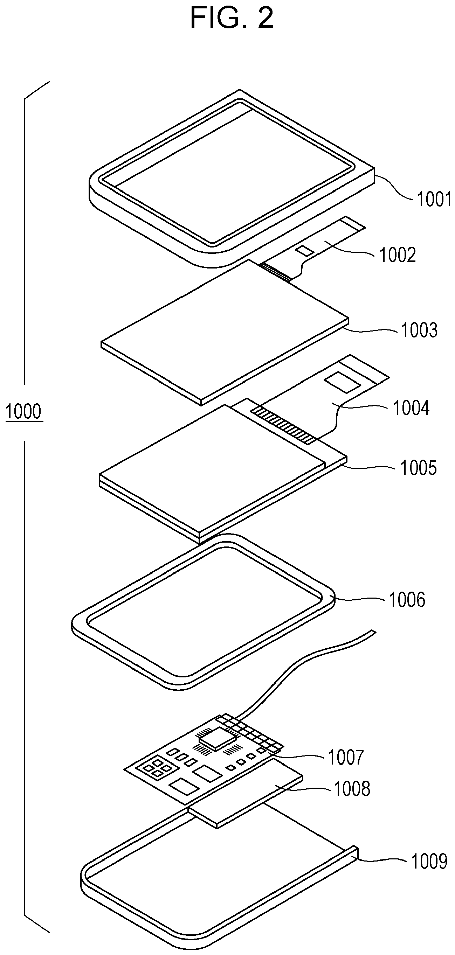

Fig. 2 is a schematic view illustrating an example of a display apparatus according to the present embodiment. A display apparatus 1000 may include a touch panel 1003, a display panel 1005, a frame 1006, a circuit substrate 1007, and a battery 1008, disposed between an upper cover 1001 and a lower cover 1009. The touch panel 1003 and the display panel 1005 are coupled to flexible printed circuits FPCs 1002 and 1004, respectively. The circuit substrate 1007 includes printed transistors. The battery 1008 need not be provided unless the display apparatus is a portable apparatus. The battery 1008 may be disposed at a different position even if the display apparatus is a portable apparatus.

The display apparatus according to the present embodiment may include a color filter having red, green, and blue portions. In the color filter, the red, green, and blue portions may be arranged in a delta arrangement.

The display apparatus according to the present embodiment may be used for the display unit of a portable terminal. In that case, the display apparatus may have both a display function and an operation function. Examples of the portable terminal include mobile phones such as smartphones, tablets, and head-mounted displays.

The display apparatus according to the present embodiment may be used for a display unit of an image pickup apparatus including an optical unit including multiple lenses and an image pickup device that receives light passing through the optical unit. The image pickup apparatus may include a display unit that displays information acquired by the image pickup device. The display unit may be a display unit exposed to the outside of the image pickup apparatus or a display unit disposed in a finder. The image pickup apparatus may be a digital camera or a digital camcorder.

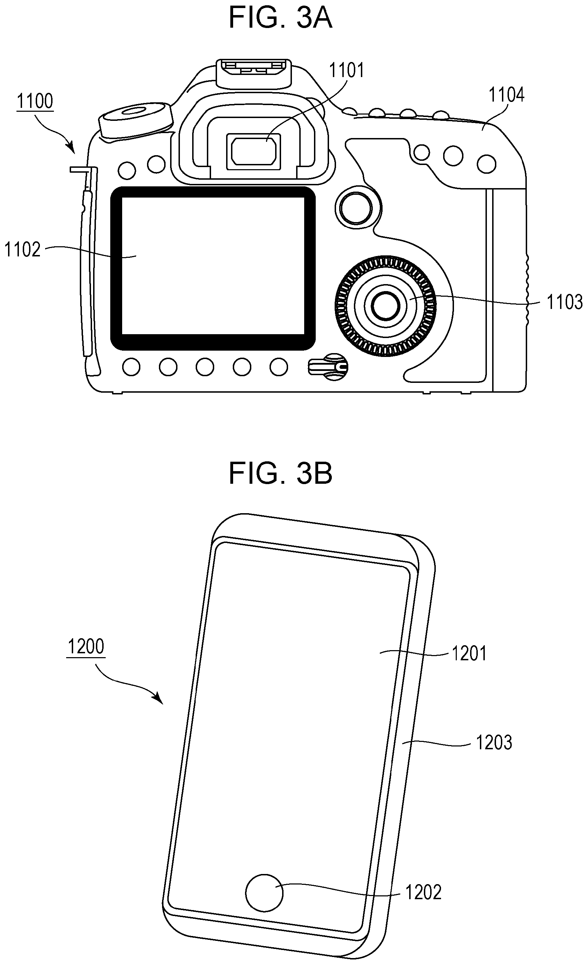

Fig. 3A is a schematic view illustrating an example of an image pickup apparatus according to the present embodiment. An image pickup apparatus 1100 may include a viewfinder 1101, a rear display 1102, an operation unit 1103, and a housing 1104. The viewfinder 1101 may include the display apparatus according to the present embodiment. In this case, the display apparatus may display environmental information, imaging instructions, and so forth in addition to an image to be captured. The environmental information may include, for example, the intensity of external light, the direction of external light, the moving speed of a subject, and the possibility that a subject is shielded by a shielding material.

The timing suitable for imaging is only for a short time. It is thus better to display the information as soon as possible. Accordingly, the display apparatus including the organic light-emitting device according to an embodiment of the present embodiment can be used. The display apparatus including the organic light-emitting device can be used more suitably than liquid crystal displays for such apparatuses required to have a high display speed.

The image pickup apparatus 1100 includes an optical unit (not illustrated). The optical unit includes multiple lenses and is configured to form an image on an image pickup device in the housing 1104. The relative positions of the multiple lenses can be adjusted to adjust the focal point. This operation can also be performed automatically. The image pickup apparatus may translate to a photoelectric conversion apparatus. Examples of an image capturing method employed in the photoelectric conversion apparatus may include a method for detecting a difference from the previous image and a method of cutting out an image from images always recorded, instead of sequentially capturing images.

Fig. 3B is a schematic view illustrating an example of an electronic apparatus according to the present embodiment. An electronic apparatus 1200 includes a display unit 1201, an operation unit 1202, and a housing 1203. The housing 1203 may accommodate a circuit, a printed circuit board including the circuit, a battery, and a communication unit. The operation unit 1202 may be a button or a touch-screen-type reactive unit. The operation unit may be a biometric recognition unit that recognizes a fingerprint to release the lock or the like. An electronic apparatus having a communication unit can also be referred to as a communication apparatus. The electronic apparatus may further have a camera function by being equipped with a lens and an image pickup device. An image captured by the camera function is displayed on the display unit. Examples of the electronic apparatus include smartphones and notebook computers.

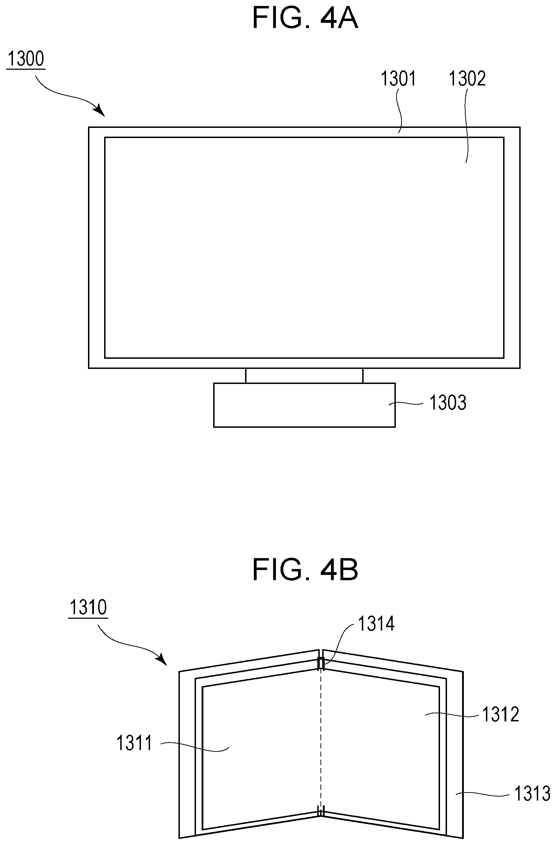

Fig. 4A is a schematic view illustrating an example of the display apparatus according to the present embodiment. Fig. 4A illustrates a display apparatus, such as a television monitor or a PC monitor. A display apparatus 1300 includes a frame 1301 and a display unit 1302. The light-emitting apparatus according to the present embodiment may be used for the display unit 1302.

The display apparatus 1300 includes a base 1303 that supports the frame 1301 and the display unit 1302. The base 1303 is not limited to the structure illustrated in Fig. 4A. The lower side of the frame 1301 may also serve as a base.

The frame 1301 and the display unit 1302 may be curved. These may have a radius of curvature of 5,000 mm or more and 6,000 mm or less.

Fig. 4B is a schematic view illustrating another example of a display apparatus according to the present embodiment. A display apparatus 1310 illustrated in Fig. 4B can be folded and is what is called a foldable display apparatus. The display apparatus 1310 includes a first display portion 1311, a second display portion 1312, a housing 1313, and an inflection point 1314. The first display portion 1311 and the second display portion 1312 may include the light-emitting apparatus according to the present embodiment. The first display portion 1311 and the second display portion 1312 may be a single, seamless display apparatus. The first display portion 1311 and the second display portion 1312 can be divided from each other at the inflection point. The first display portion 1311 and the second display portion 1312 may display different images. Alternatively, a single image may be displayed in the first and second display portions.

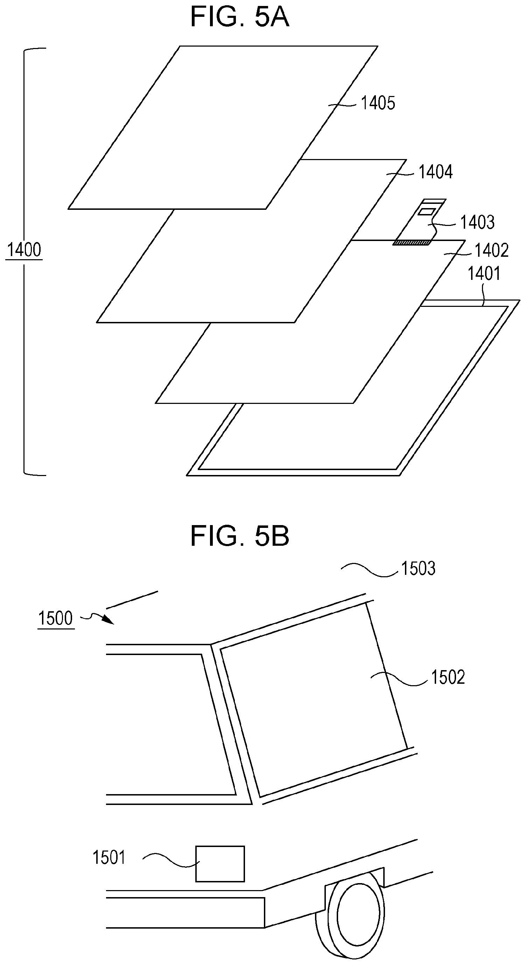

Fig. 5A is a schematic view illustrating an example of a lighting apparatus according to the present embodiment. A lighting apparatus 1400 may include a housing 1401, a light source 1402, a circuit board 1403, an optical film 1404, and a light diffusion unit 1405. The light source may include an organic light-emitting device according to the present embodiment. The optical film may be a film that improves the color rendering properties of the light source. The light diffusion unit can effectively diffuse light from the light source to deliver the light to a wide range when used for illumination and so forth. The optical film and the light diffusion unit may be disposed at the light emission side of the lighting apparatus. A cover may be disposed at the outermost portion, as needed.

The lighting apparatus is, for example, an apparatus that lights a room. The lighting apparatus may emit light of white, neutral white, or any color from blue to red. A light control circuit that controls the light may be provided.

The lighting apparatus may include the organic light-emitting device according to an embodiment of the present invention and a power supply circuit coupled thereto. The power supply circuit is a circuit that converts an AC voltage into a DC voltage. The color temperature of white is 4,200 K, and the color temperature of neutral white is 5,000 K. The lighting apparatus may include a color filter.

The lighting apparatus according to the present embodiment may include a heat dissipation unit. The heat dissipation unit is configured to release heat in the device to the outside of the device and is composed of, for example, a metal having a high specific heat and liquid silicone.

Fig. 5B is a schematic view illustrating an automobile as an example of a moving object according to the present embodiment. The automobile includes a tail lamp, which is an example of lighting units. An automobile 1500 includes a tail lamp 1501 and may be configured to light the tail lamp when a brake operation or the like is performed.

The tail lamp 1501 may include an organic light-emitting device according to the present embodiment. The tail lamp may include a protective member that protects the organic light-emitting device. The protective member may be composed of any transparent material having high strength to some extent and can be composed of, for example, polycarbonate. The polycarbonate may be mixed with, for example, a furandicarboxylic acid derivative or an acrylonitrile derivative.

The automobile 1500 may include an automobile body 1503 and windows 1502 attached thereto. The windows may be transparent displays if the windows are not used to check the front and back of the automobile. The transparent displays may include an organic light-emitting device according to the present embodiment. In this case, the components, such as the electrodes, of the organic light-emitting device are formed of transparent members.

The moving object according to the present embodiment may be, for example, a ship, an aircraft, or a drone. The moving object may include a body and a lighting unit attached to the body. The lighting unit may emit light to indicate the position of the body. The lighting unit includes the organic light-emitting device according to the present embodiment.

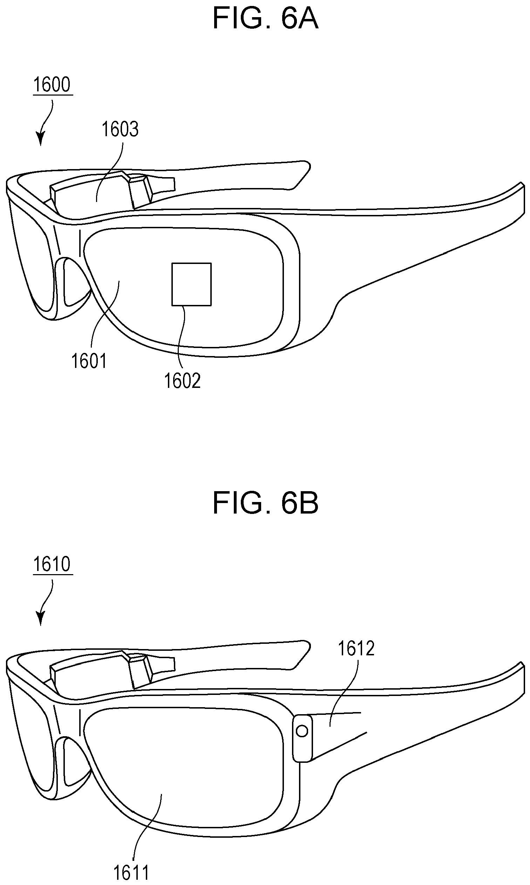

Examples of applications of the display apparatuses of the above embodiments will be described with reference to Figs. 6A and 6B. The display apparatuses can be used for systems that can be worn as wearable devices, such as smart glasses, head-mounted displays (HMDs), and smart contacts. An image pickup and display apparatus used in such an example of the applications has an image pickup apparatus that can photoelectrically convert visible light and a display apparatus that can emit visible light.

A wearable device may include the organic light-emitting device according to the present embodiment, an optical unit configured to collect light emitted from the organic light-emitting device, and a projection unit configured to project light passing through the optical unit. The optical unit includes multiple lenses, and condenses and refracts light emitted from the organic light-emitting device. The projection unit may be composed of, for example, glass or plastic. The projection unit can have higher optical transparency, but need not be colorless.

Fig. 6A illustrates glasses 1600 (smart glasses) according to an example of applications. An image pickup apparatus 1602, such as a complementary metal-oxide semiconductor (CMOS) sensor or a single-photon avalanche diode (SPAD), is provided on a front side of a lens 1601 of the glasses 1600. The display apparatus according to any of the above-mentioned embodiments is provided on the back side of the lens 1601.

The glasses 1600 further include a control unit 1603. The control unit 1603 functions as a power source that supplies electric power to the image pickup apparatus 1602 and the display apparatus according to any of the embodiments. The control unit 1603 controls the operation of the image pickup apparatus 1602 and the display apparatus. The lens 1601 has an optical system for focusing light on the image pickup apparatus 1602.

Fig. 6B illustrates glasses 1610 (smart glasses) according to an example of applications. The glasses 1610 include a control unit 1612. The control unit 1612 includes an image pickup apparatus corresponding to the image pickup apparatus 1602 and a display apparatus. A lens 1611 includes an optical system configured to project light emitted from the display apparatus in the control unit 1612. An image is projected onto the lens 1611. The control unit 1612 functions as a power source that supplies electric power to the image pickup apparatus and the display apparatus and controls the operation of the image pickup apparatus and the display apparatus. The control unit may include a gaze detection unit that detects the gaze of a wearer. Infrared light may be used for gaze detection.

An infrared light-emitting unit emits infrared light to an eyeball of a user who is gazing at a displayed image. An image of the eyeball is captured by detecting the reflected infrared light from the eyeball with an image pickup unit having light-receiving elements. The deterioration of image quality is reduced by providing a reduction unit configured to reduce light from the infrared light-emitting unit to the display unit when viewed in plan.

The user's gaze at the displayed image is detected from the image of the eyeball captured with the infrared light. Any known method can be used to the gaze detection using the captured image of the eyeball. As an example, a gaze detection method based on a Purkinje image of the reflection of irradiation light on a cornea can be used.

More specifically, the gaze detection process is based on a pupil-corneal reflection method. Using the pupil-corneal reflection method, the user's gaze is detected by calculating a gaze vector representing the direction (rotation angle) of the eyeball based on the image of the pupil and the Purkinje image contained in the captured image of the eyeball.

A display apparatus according to an embodiment of the present invention may include an image pickup apparatus including light-receiving elements, and may control an image displayed on the display apparatus based on the gaze information of the user from the image pickup apparatus.

Specifically, in the display apparatus, a first display area at which the user gazes and a second display area other than the first display area are determined on the basis of the gaze information. The first display area and the second display area may be determined by the control unit of the display apparatus or may be determined by receiving those determined by an external control unit. In the display area of the display apparatus, the display resolution of the first display area may be controlled to be higher than the display resolution of the second display area. That is, the resolution of the second display area may be lower than that of the first display area.

The display area includes a first display area and a second display area different from the first display area. Based on the gaze information, an area of higher priority is determined from the first display area and the second display area. The first display area and the second display area may be determined by the control unit of the display apparatus or may be determined by receiving those determined by an external control unit. The resolution of an area of higher priority may be controlled to be higher than the resolution of an area other than the area of higher priority. In other words, the resolution of an area of a relatively low priority may be low.

Artificial intelligence (AI) may be used to determine the first display area or the high-priority area. The AI may be a model configured to estimate the angle of gaze from the image of the eyeball and the distance to a target object located in the gaze direction, using the image of the eyeball and the actual direction of gaze of the eyeball in the image as teaching data. The AI program may be stored in the display apparatus, the image pickup apparatus, or an external apparatus. When the AI program is stored in the external apparatus, the AI program is transmitted to the display apparatus via communications.

In the case of controlling the display based on visual detection, smart glasses that further include an image pickup apparatus that captures an external image can be used. The smart glasses can display the captured external information in real time.

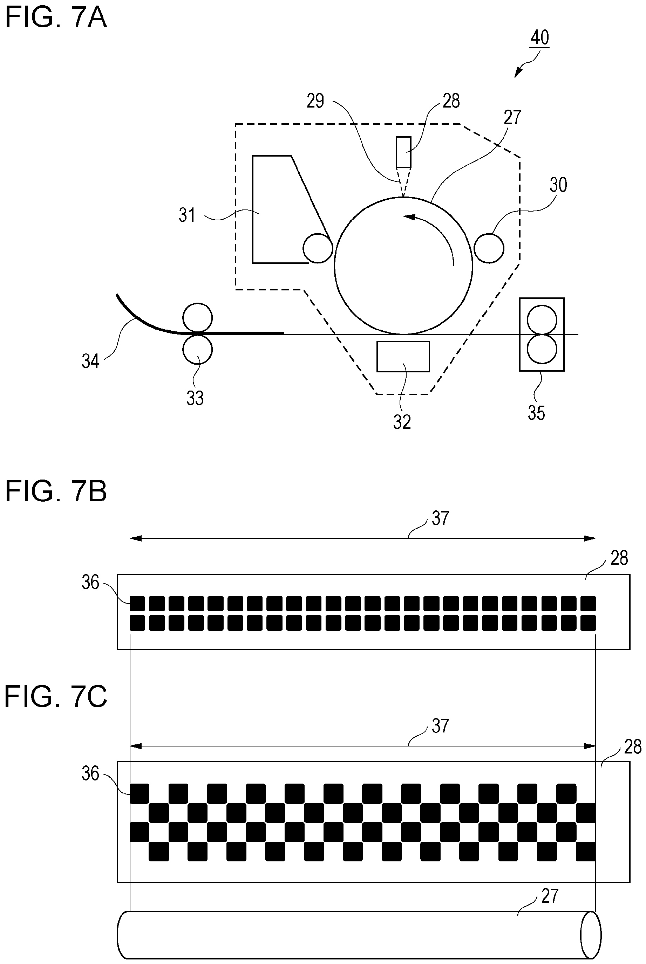

Figs. 7A, 7B, and 7C illustrate an image-forming apparatus according to an embodiment of the present invention. Fig. 7A is a schematic view of an image-forming apparatus 36 according to an embodiment of the present invention. The image-forming apparatus includes a photoconductor, an exposure light source, a developing unit, a charging unit, a transfer unit, a transport roller, and a fusing unit.

The irradiation of light 29 is performed from the exposure light source 28 to form an electrostatic latent image on the surface of the photoconductor 27. This exposure light source includes an organic light-emitting device according to an embodiment of the present invention. The developing unit 31 contains, for example, a toner. The charging unit 30 charges the photoconductor. The transfer unit 32 transfers the developed image to a recording medium 34. The transport roller 33 transports the recording medium 34. The recording medium 34 is paper, for example. The fusing unit 35 fixes the image formed on the recording medium.

Figs. 7B and 7C are each a schematic view illustrating a state in which multiple light-emitting portions 38 are arranged on a long substrate in the exposure light source 28. Arrows 37 are parallel to the axis of the photoconductor and each represent the row direction in which the organic light-emitting devices are arranged. The row direction is the same as the direction of the axis on which the photoconductor 27 rotates. This direction can also be referred to as the long-axis direction of the photoconductor.

Fig. 7B illustrates a configuration in which the light-emitting portions are arranged in the long-axis direction of the photoconductor. Fig. 7C is different from Fig. 7B in that the light-emitting portions are arranged alternately in the row direction in a first row and a second row. The first row and the second row are located at different positions in the column direction.

In the first row, the multiple light-emitting portions are spaced apart. The second row has the light-emitting portions at positions corresponding to the positions between the light-emitting portions in the first row. In other words, the multiple light-emitting portions are also spaced apart in the column direction.

The arrangement in Fig. 7C can be rephrased as, for example, a lattice arrangement, a staggered arrangement, or a checkered pattern.

As described above, the use of an apparatus including the organic light-emitting device according to the present embodiment enables a stable display with good image quality even for a long time.

### EXAMPLES

Combinations of components in compositions according to embodiments of the present invention and comparative configurations are presented in Table 1 together with calculated values of their LUMO levels. Combinations of compounds are presented as part of the present invention, and the present invention is not limited thereto.

**Table 1**

| | | Organic compound AB | Organic compound AA | Light-emitting compound | Emission color | Operating lifetime |
|---|---|---|---|---|---|---|
| Inventive configuration 1-1 | Molecular structure | 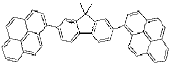 | 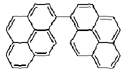 | 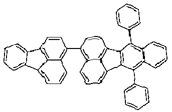 | blue | 1.3 |
| | LUMO | -1.67 | -1.69 | | | |
| Comparative configuration 1-1 | Molecular structure | 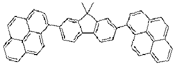 | | | | 1 |
| | LUMO | -1.67 | | | | |
| Inventive configuration 1-2 | Molecular structure | 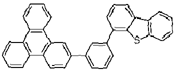 | 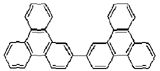 | 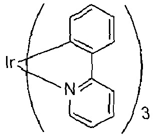 | green | 1.2 |
| | LUMO | -1.20 | -1.35 | | | |
| Comparative configuration 1-2 | Molecular structure | 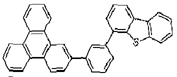 | | | | 1 |
| | LUMO | -1.20 | | | | |
| Inventive configuration 1-3 | Molecular structure | 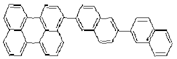 | 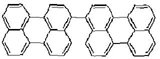 | 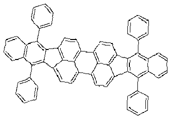 | red | 1.5 1 |
| | LUMO | -1.96 | -2.00 | | | |
| Comparative configuration 1-3 | Molecular structure | 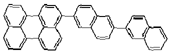 | | | | |
| | LUMO | -1.96 | | | | |
| | | Organic compound AB | Comparative compound | | | |
| Comparative configuration 1-4 | Molecular structure | 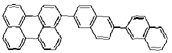 | 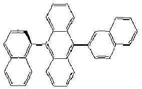 | 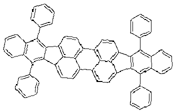 | red | 0.4 |
| | LUMO | -1.96 | -1.64 | | | |

Inventive configuration 1-1 includes the organic compound AB and the organic compound AA, whereas Comparative configuration 1-1 does not include the organic compound AA. Comparative configurations 1-2 and 1-3 also differ from Inventive configurations 1-2 and 1-3, respectively, in that they do not contain the organic compound AA. Comparative configuration 1-4 includes the organic compound AB and a comparative compound. The comparative compound is largely different in structure from the organic compound AB and thus does not provide the effects according to an embodiment of the present invention.

As the molecular orbital calculation method, the density functional theory (DFT), which is widely used at present, was used with the B3LYP functional and 6-31G* as the basis function. The molecular orbital calculation method was performed using Gaussian 09 (Gaussian 09, Revision C.01, M.J. Frisch, G.W. Trucks, H.B. Schlegel, G.E. Scuseria, M.A. Robb, J.R. Cheeseman, G. Scalmani, V. Barone, B. Mennucci, G.A. Petersson, H. Nakatsuji, M. Caricato, X. Li, H.P. Hratchian, A.F. Izmaylov, J. Bloino, G. Zheng, J.L. Sonnenberg, M. Hada, M. Ehara, K. Toyota, R. Fukuda, J. Hasegawa, M. Ishida, T. Nakajima, Y. Honda, O. Kitao, H. Nakai, T. Vreven, J.A. Montgomery, Jr., J.E. Peralta, F. Ogliaro, M. Bearpark, J.J. Heyd, E. Brothers, K.N. Kudin, V.N. Staroverov, T. Keith, R. Kobayashi, J. Normand, K. Raghavachari, A. Rendell, J.C. Burant, S.S. Iyengar, J. Tomasi, M. Cossi, N. Rega, J.M. Millam, M. Klene, J.E. Knox, J.B. Cross, V. Bakken, C. Adamo, J. Jaramillo, R. Gomperts, R.E. Stratmann, O. Yazyev, A.J. Austin, R. Cammi, C. Pomelli, J.W. Ochterski, R.L. Martin, K. Morokuma, V.G. Zakrzewski, G.A. Voth, P. Salvador, J.J. Dannenberg, S. Dapprich, A.D. Daniels, O. Farkas, J.B. Foresman, J.V. Ortiz, J. Cioslowski, and D.J. Fox, Gaussian, Inc., Wallingford CT, 2010), which is widely used at present.

### Example 1

An organic light-emitting device having a bottom-emission structure was produced in which an anode, a hole injection layer, a hole transport layer, an electron-blocking layer, a light-emitting layer, a hole-blocking layer, an electron transport layer, an electron injection layer, and a cathode were sequentially formed on a substrate.

An ITO film was formed on a glass substrate and subjected to desired patterning to form an ITO electrode (anode). The ITO electrode had a thickness of 100 nm. The substrate on which the ITO electrode had been formed in this way was used as an ITO substrate in the following steps. Next, vacuum deposition was performed by resistance heating in a vacuum chamber at 1.33 × 10⁻⁴ Pa to successively form organic compound layers and an electrode layer presented in Table 2 on the ITO substrate. Here, the opposing electrode (metal electrode layer, cathode) had an electrode area of 3 mm².

**Table 2**

| | Material | | Thickness (nm) |
|---|---|---|---|
| Cathode | Al | | 100 |
| Electron injection layer (EIL) | LiF | | 1 |
| Electron transport layer (ETL) | ET7 | | 20 |
| Hole-blocking layer (HBL) | ET12 | | 20 |
| Light-emitting layer (EML) | First host material (Organic compound AB) | EM4: 97% | 20 |
| | Second host material (Organic compound AA) | AA1: 1% | |
| | Light-emitting compound | BD8: 2% | |
| Electron-blocking layer (HBL) | HT15 | | 15 |
| Hole transport layer (HTL) | HT2 | | 30 |
| Hole injection layer (HIL) | HT16 | | 5 |

The characteristics of the resulting device were measured and evaluated. The emission color of the light-emitting device was a good blue color. The device was subjected to a continuous operation test at a current density of 100 mA/cm². The time when the percentage of luminance degradation reached 5% was measured. When the time when the percentage of luminance degradation of Comparative example 1 reached 5% was defined as 1.0, the percentage ratio of luminance degradation in this example was determined and found to be 1.5.

With regard to measurement instruments, in this example, the current-voltage characteristics were measured with a Hewlett-Packard 4140B microammeter, and the luminance was measured with a Topcon BM7.

### Examples 2 to 10 and Comparative Example 1 to 6

Organic light-emitting devices in Examples 2 to 10 were produced in the same manner as in Example 1, except that compounds listed in Table 3 were used as appropriate. The characteristics of the resulting devices were measured and evaluated as in Example 1. Table 3 presents the measurement results.

**Table 3**

| | HIL | HTL | EBL | EML | | | HBL | ETL | Percentage ratio of luminance degradation |
|---|---|---|---|---|---|---|---|---|---|
| | | | | First host /compound AB | Second host /compound AA | Light-emitting compound | | | |
| Example 2 | HT16 | HT2 | HT15 | EM4 97.5% | AA1 0.5% | BD8 2% | ET12 | ET7 | 1.5 |
| Example 3 | HT16 | HT2 | HT15 | EM4 88% | AA1 10% | BD8 2% | ET12 | ET7 | 1.5 |
| Example 4 | HT16 | H T2 | HT15 | EM4 52% | AA1 46% | BD8 2% | ET12 | ET7 | 1.4 |
| Comparative example 1 | HT16 | HT2 | HT15 | EM4 98% | | BD8 2% | ET12 | ET7 | 1 |
| Example 5 | H T16 | HT3 | H T19 | EM7 97% | EM8 1% | BD8 2% | ET11 | ET7 | 1.4 |
| Comparative example 2 | HT16 | HT3 | HT19 | EM7 98% | | BD8 2% | ET11 | ET7 | 1 |
| Example 6 | HT16 | HT10 | HT6 | EM5 98.5% | AA5 0.5% | BD3 1% | ET12 | ET2 | 1.2 |
| Comparative example 3 | HT16 | HT10 | HT6 | EM5 99% | | BD3 1% | ET12 | ET2 | 1 |
| Example 7 | HT16 | HT2 | HT19 | EM9 95% | AA6 3% | BD10 2% | ET10 | ET7 | 1.4 |
| Comparative example 4 | HT16 | HT2 | HT19 | EM9 98% | | BD10 2% | ET10 | ET7 | 1 |
| Example 8 | HT16 | HT10 | HT4 | EM41 97% | AA1 1% | BD8 2% | ET10 | ET7 | 1.5 |
| Comparative example 5 | HT16 | HT10 | HT4 | EM41 98% | | BD8 2% | ET10 | ET7 | 1 |
| Example 9 | HT16 | HT2 | HT15 | EM1 97.5% | AA1 0.5% | BD10 2% | ET11 | ET2 | 1.6 |
| Example 10 | HT16 | HT2 | HT15 | EM1 97% | AA1 1% | BD10 2% | ET11 | ET2 | 1.4 |
| Comparative example 6 | HT16 | HT2 | HT15 | EM1 97.98% | AA1 0.02% | BD10 2% | ET11 | ET2 | 1 |

Table 3 indicates that the percentage ratios of luminance degradation in Examples 2 to 10 were more than 1.0 with respect to the respective percentage ratios of luminance degradation in Comparative examples 1 to 6, and the organic light-emitting devices according to the embodiments of the present invention had longer lifetimes. The use of the organic compound AB and the organic compound AA according to an embodiment of the present invention as host materials for the light-emitting layer provides a good operating lifetime. The light-emitting devices presented in Table 3 all exhibited good blue emission.

### Examples 11 to 16 and Comparative Examples 7 to 12

Organic light-emitting devices in Examples 11 to 16 were produced in the same manner as in Example 1, except that compounds listed in Table 4 were used as appropriate. The characteristics of the resulting devices were measured and evaluated as in Example 1. Table 4 presents the measurement results. The emission color of each of the light-emitting devices was a good green color. The percentage ratio of luminance degradation in each of the examples refers to a relative ratio when the time at which the percentage of luminance degradation of a corresponding one of the comparative examples reaches 5% is defined as 1.0.

**Table 4**

| | HIL | HTL | EBL | EML | | | HBL | ETL | Percentage ratio of luminance degradation |
|---|---|---|---|---|---|---|---|---|---|
| | | | | First host /compound AB | Second host /compound AA | Light-emitting compound | | | |
| Example 11 | HT16 | HT2 | H T4 | EM13 85% | AA2 2% | GD10 12% | ET12 | ET7 | 1.4 |
| Comparative example 7 | HT16 | HT2 | HT4 | EM13 88% | | GD10 12% | ET12 | ET7 | 1 |
| Example 12 | HT16 | HT2 | H T19 | EM14 87% | AA2 3% | GD13 10% | ET12 | ET7 | 1.5 |
| Comparative example 8 | HT16 | HT2 | HT19 | EM14 90% | | GD13 10% | ET12 | ET7 | 1 |
| Example 13 | HT16 | HT2 | HT15 | EM1 97% | AA1 1% | GD6 2% | ET10 | ET7 | 1.6 |
| Comparative example 9 | HT16 | HT2 | HT15 | EM1 98% | | GD6 2% | ET10 | ET7 | 1 |
| Example 14 | HT16 | HT2 | HT4 | EM28 78% | AA7 10% | GD10 12% | ET12 | ET7 | 1.2 |
| Comparative example 10 | HT16 | HT2 | HT4 | EM28 88% | | GD10 12% | ET12 | ET7 | 1 |
| Example 15 | HT16 | HT2 | HT4 | ET27 86% | AA8 2% | GD10 12% | ET12 | ET7 | 1.2 |
| Comparative example 11 | HT16 | HT2 | HT4 | ET27 88% | | GD10 12% | ET12 | ET7 | 1 |
| Example 16 | HT16 | HT2 | HT15 | EM4 98% | AA1 1% | GD4 1% | ET10 | ET7 | 1.5 |
| Comparative example 12 | HT16 | HT2 | HT15 | EM4 99% | | GD4 1% | ET10 | ET7 | 1 |

Table 4 indicates that the percentage ratios of luminance degradation in Examples 11 to 16 were more than 1.0 with respect to the respective percentage ratios of luminance degradation in Comparative examples 7 to 12, and the organic light-emitting devices according to the embodiments of the present invention had longer lifetimes. The use of the organic compound AB and the organic compound AA according to an embodiment of the present invention as host materials for the light-emitting layer provides a good operating lifetime. Each of the light-emitting devices presented in Table 4 exhibited good green emission.

### Example 17 to 26 and Comparative Example 13 to 19

Organic light-emitting devices in Examples 17 to 26 were produced in the same manner as in Example 1, except that compounds listed in Table 5 were used as appropriate, the EBL was eliminated, each HTL had a thickness of 37 nm, and each HBL had a thickness of 34 nm. The characteristics of the resulting devices were measured and evaluated as in Example 1. Table 5 presents the measurement results. The emission color of each of the organic light-emitting devices was a good red color. The percentage ratio of luminance degradation in each of the examples refers to a relative ratio when the time at which the percentage of luminance degradation of a corresponding one of the comparative examples reaches 5% is defined as 1.0.

**Table 5**

| | HIL | HTL | EML | | | HBL | ETL | Percentage ratio of luminance degradation |
|---|---|---|---|---|---|---|---|---|
| | | | First host /compound AB | Second host /compound AA | Light-emitting compound | | | |
| Example 17 | HT16 | HT3 | EM25 98.5% | AA4 1% | RD4 0.5% | EM1 | ET7 | 1.5 |
| Comparative example 13 | HT16 | HT3 | EM25 99.5% | | RD4 0.5% | EM1 | ET7 | 1 |
| Example 18 | HT16 | HT3 | EM22 97.8% | AA3 2% | RD3 0.2% | EM1 | ET7 | 1.5 |
| Example 19 | HT16 | HT3 | EM22 89.8% | AA3 10% | RD3 0.2% | EM1 | ET7 | 1.6 |
| Comparative example 14 | HT16 | HT3 | EM22 99.8% | | RD3 0.2% | EM1 | ET7 | 1 |
| Example 20 | HT16 | HT3 | EM23 97.8% | AA3 2% | RD1 0.2% | EM1 | ET7 | 1.5 |
| Comparative example 15 | HT16 | HT3 | EM23 99.8% | | RD1 0.2% | EM1 | ET7 | 1 |
| Example 21 | HT16 | HT3 | EM23 97.8% | AA3 2% | RD2 0.2% | EM1 | ET7 | 1.6 |
| Example 22 | HT16 | HT3 | EM23 99.3% | AA3 0.5% | RD2 0.2% | EM1 | ET7 | 1.7 |
| Comparative example 16 | HT16 | HT3 | EM23 99.8% | | RD2 0.2% | EM1 | ET7 | 1 |
| Example 23 | HT16 | HT7 | EM23 97.5% | AA3 2% | RD4 0.5% | ET12 | ET7 | 1.8 |
| Comparative example 17 | HT16 | HT7 | EM23 99.5% | | RD4 0.5% | ET12 | ET7 | 1 |
| Example 24 | HT16 | HT7 | EM43 95% | AA9 1% | RD5 4% | ET12 | ET7 | 1.2 |
| Example 25 | H T16 | HT7 | EM43 66% | AA9 30% | RD5 4% | ET12 | ET7 | 1.2 |
| Comparative example 18 | HT16 | HT7 | EM43 96% | | RD5 4% | ET12 | ET7 | 1 |
| Example 26 | HT16 | HT7 | EM13 95% | AA2 1% | RD7 4% | ET12 | ET7 | 1.5 |
| Comparative example 19 | HT16 | HT7 | EM13 96% | | RD7 4% | ET12 | ET7 | 1 |

Table 5 indicates that the percentage ratios of luminance degradation in Examples 17 to 26 were more than 1.0 with respect to the respective percentage ratios of luminance degradation in Comparative examples 13 to 19, and the organic light-emitting devices according to the embodiments of the present invention had longer lifetimes. The use of the organic compound AB and the organic compound AA according to an embodiment of the present invention as host materials for the light-emitting layer provides a good operating lifetime. Each of the light-emitting devices presented in Table 5 exhibited good red emission.

As described above, the use of the composition according to an embodiment of the present invention can provide the device having high efficiency and excellent durability characteristics.

According to an embodiment of the present invention, it is possible to provide the composition that improves the characteristics of an organic light-emitting device by using the relationship between the monomer and the multimer.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

The present invention provides a composition containing an organic compound AB and an organic compound AA different from the organic compound AB. The organic compound AB is represented by the following general formula [1] and has a fused polycyclic structure A with two or more rings and a ring structure B different from the fused polycyclic structure A. The organic compound AA has the fused polycyclic structure A and is represented by the following general formula [2],

A-B [1]

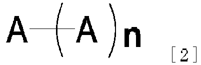

In general formulae [1] and [2], each of the fused polycyclic structures A may have, as a substituent, a deuterium atom, a substituted or unsubstituted alkyl group, a substituted or unsubstituted aryl group, or a substituted or unsubstituted heteroaryl group.

## Claims

1. A composition, comprising:
an organic compound AB; and
an organic compound AA different from the organic compound AB,
wherein the organic compound AB is represented by the following general formula [1] and has a fused polycyclic structure A with two or more rings and a ring structure B different from the fused polycyclic structure A,
the organic compound AA has the fused polycyclic structure A and is represented by the following general formula [2],
A-B [1] 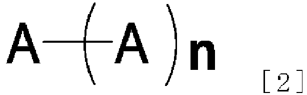
where in general formulae [1] and [2], each of the fused polycyclic structures A optionally has, as a substituent, a deuterium atom, a substituted or unsubstituted alkyl group, a substituted or unsubstituted aryl group, or a substituted or unsubstituted heteroaryl group, and in general formula [2], n represents the number of fused polycyclic structures A.

2. The composition according to Claim 1, wherein the fused polycyclic structure A is a fused polycyclic structure with four or more rings.

3. The composition according to Claim 2, wherein the fused polycyclic structure A is selected from the following structures: 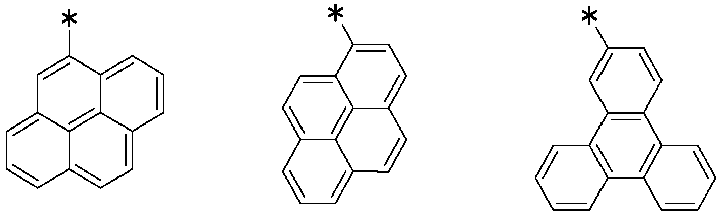 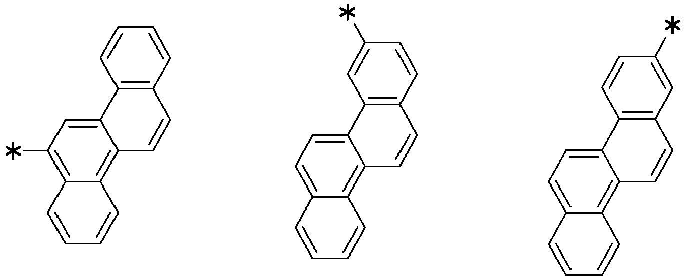 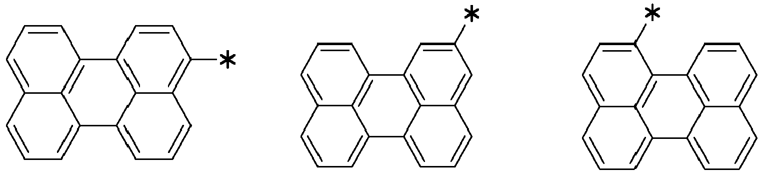 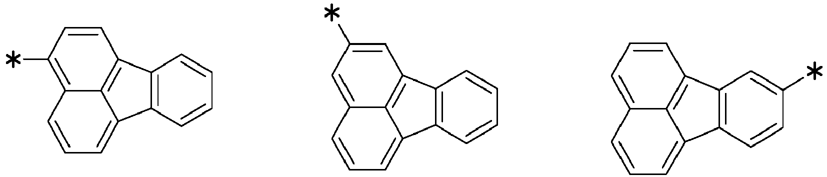 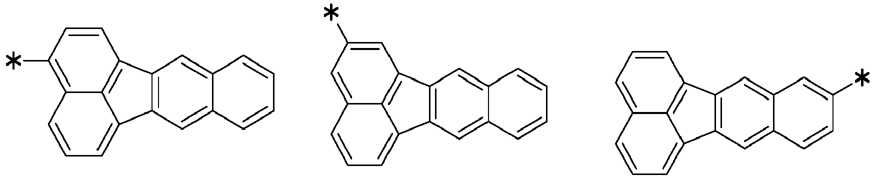 where each * represents a binding position.

4. The composition according to Claim 3, wherein the fused polycyclic structure A is a perylene structure, a triphenylene structure, or a pyrene structure.

5. The composition according to any one of Claims 1 to 4, wherein an element contained in the fused polycyclic structure A is an element selected from the group consisting of a hydrogen atom, a deuterium atom, a nitrogen atom, an oxygen atom, and a sulfur atom.

6. The composition according to any one of Claims 1 to 5, wherein the ring structure B is an aryl group with four or less rings.

7. The composition according to Claim 5, wherein the ring structure B is a heteroaryl group with four or less rings.

8. The composition according to Claim 6, wherein the ring structure B is selected from the group consisting of a phenyl group, a naphthyl group, and a fluorenyl group.

9. The composition according to any one of Claims 1 to 8, wherein a proportion by weight of the organic compound AA is more than 0.02% by weight and 10% or less by weight based on a total weight of the organic compound AB and the organic compound AA.

10. The composition according to any one of Claims 1 to 9, wherein n is 1.

11. An organic light-emitting device (26), comprising:
a first electrode;
a second electrode; and
an organic compound layer (4) disposed between the first electrode and the second electrode,
wherein the organic compound layer (4) contains the composition according to any one of Claims 1 to 10.

12. The organic light-emitting device (26) according to Claim 11, wherein the organic compound layer (4) is a light-emitting layer.

13. The organic light-emitting device (26) according to Claim 11, wherein the light-emitting layer contains:
a light-emitting compound,
the organic compound AB, and
the organic compound AA,
wherein the organic compound AB, the organic compound AA, and the light-emitting compound are in decreasing order of energy gaps thereof.

14. The organic light-emitting device (26) according to Claim 17, wherein the light-emitting layer contains:
a light-emitting compound,
the organic compound AB, and
the organic compound AA,
wherein the organic compound AB, the organic compound AA, and the light-emitting compound are in decreasing order of proportions by weight thereof.

15. The organic light-emitting device (26) according to Claim 12, further comprising:
a second light-emitting layer different from the light-emitting layer,
wherein the second light-emitting layer (44) contains a second composition different from the composition, the second composition contains:
an organic compound CD, and
an organic compound CC different from the organic compound CD,
wherein the organic compound CD is represented by the following general formula [4] and has a fused polycyclic structure C with two or more rings and a ring structure D different from the fused polycyclic structure C,
the organic compound CC is represented by the following general formula [5] and has the fused polycyclic structure C,
C-D [4] 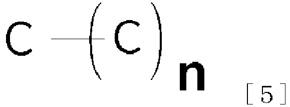
where in general formulae [4] and [5], each of the fused polycyclic structure C optionally has, as a substituent, a deuterium atom, a substituted or unsubstituted alkyl group, a substituted or unsubstituted aryl group, or a substituted or unsubstituted heteroaryl group.

16. The organic light-emitting device (26) according to Claim 15, wherein a proportion by weight of the organic compound CC is more than 0.1% by weight and 10% or less by weight based on a total weight of the organic compound CD and the organic compound CC.

17. A display apparatus (100), comprising:
multiple pixels,
at least one of the multiple pixels including:
the organic light-emitting device (26) according to any one of Claims 11 to 16, and
a transistor coupled to the organic light-emitting device.

18. A photoelectric conversion apparatus, comprising:
an optical unit including multiple lenses;
an image pickup device configured to receive light passing through the optical unit; and
display means arranged to display an image captured by the image pickup device,
wherein the display means includes the organic light-emitting device (26) according to any one of Claims 11 to 16.

19. A lighting apparatus (1400), comprising:
a light source (1402) including the organic light-emitting device (26) according to any one of Claims 11 to 16; and
light diffusion means (1405) or an optical film (1404) configured to transmit light emitted from the light source (1402).

20. A head-mounted display, comprising:
the organic light-emitting device (26) according to any one of Claims 11 to 16; and
optical means arranged to collect light emitted from the organic light-emitting device (26).
